# EUROPEAN PATENT APPLICATION

(11) **EP 4 578 953 A1**
(43) Date of publication of application: **02.07.2025**
(21) Application number: 23857405.7
(22) Date of filing: 24.08.2023
(51) Int. Cl.: C12P 19/00, C12P 19/44, C12N 9/10

(54) **COMPOSITION PRODUCTION METHOD, OLIGOSACCHARIDE-CONTAINING COMPOSITION OBTAINED THEREBY, AND UTILIZATION OF SAID METHOD AND COMPOSITION**

(30) Priority: 25.08.2022 JP 2022134443; 25.08.2022 JP 2022134446
(71) Applicant: MEIJI CO., LTD, Chuo-ku Tokyo 104-8306 (JP)
(72) Inventor: ISHIDA Junya, Hachioji-shi, Tokyo 192-0919 (JP); ICHIKAWA Ayumi, Hachioji-shi, Tokyo 192-0919 (JP)
(74) Representative: TBK
(86) International application number: PCT/JP2023/030454
(87) International publication number: WO 2024/043297

(57) **Abstract**

An object is to provide a method for effectively utilizing byproducts of the production of dairy products. Another object is to provide a method for producing galactosylkojibiose having a superior prebiotic ability. Another object is to provide a solution or composition comprising galactosylkojibiose, wherein at least 80% of galactosylkojibiose is maintained even after heated. A method for producing a composition comprising an oligosaccharide, the method comprising the step of allowing a glucansucrase to act on a composition comprising lactose and sucrose to obtain a composition comprising galactosylkojibiose is provided. It is preferable to use a lactic acid bacterium that constitutively expresses a membrane-bound type glucansucrase. There are provided a solution containing galactosylkojibiose, which has a pH lower than 7, and a composition comprising galactosylkojibiose, wherein when the composition is made into a solution having a solid content concentration of 10 to 80%, the solution has a pH lower than 7.

## Description

### Technical Field

The present invention relates to a method for producing a composition comprising an oligosaccharide such as galactosylkojibiose, a composition comprising an oligosaccharide obtained thereby, and uses thereof.

### Background Art

Lactose accounts for about 99.8% of the carbohydrates in milk and is relatively abundantly obtained as a byproduct in the manufacturing process of dairy products. Regarding the use of lactose, Patent document 1, of which invention relates to the production of oligosaccharides, describes a method for producing a mixture of oligosaccharides containing galactose in the molecules thereof, which comprises allowing a glycosyltransferase to act on a mixture of galactose and/or lactose as a sugar acceptor and a starch partial hydrolysate as a sugar donor. This reference describes that a culture supernatant of *Bacillus macerans* (IFO 8490, IAM 1227), or the like can be used as the glycosyltransferase.

As glycosyltransferase, there are known lactases, which use lactose as a sugar donor and sugar acceptor, enzymes that use lactose as a sugar acceptor and other sugars as a sugar donor, and so forth. Glucansucrase, one of the latter enzymes, has an activity to transfer glucose to various sugars using sucrose as a sugar donor. It is known that certain microorganisms express glucansucrase, and there are several reports on uses thereof. For example, Patent document 2 describes a reaction composition containing at least water, sucrose, an α-glucan substrate, and a polypeptide capable of forming at least one α-1,2-branched chain from the α-glucan substrate, and mentions glucosyltransferases derived from microorganisms such as *Fructobacillus tropaeoli* as examples of that polypeptide. Patent document 3 describes use of glycosyltransferase to improve the texture of fermented milk-based products, and describes a glucosyltransferase enzyme derived from *Streptococcus salivarius* SK126 and its mutants as the glycosyltransferase.

In addition, lactic acid bacteria that express glucansucrase are known. For example, Non-patent document 1 describes use of *Leuconostoc mesenteroides* to obtain trisaccharides from a sucrose-lactose mixed sugar culture medium. Further, Non-patent document 2, which relates to glucan synthesis in the *Lactobacillus* bacteria, describes isolation and characterization of glucansucrase genes and enzymes derived from six different strains of *Lactobacillus* bacteria, as well as the glucans produced thereby. Non-patent document 3 relates to production of glucan with a glucansucrase derived from a strain of *Lactobacillus satsumensis* isolated from a fermented beverage starter culture. This reference describes production by this strain of water-soluble dextrans composed mainly of α-(1→6)-linked D-glucose units from sucrose and water-insoluble glucans comprising D-glucose units linked through both α-(1→6) and α-(1→3) linkages. Furthermore, Non-patent document 4 relates to two types of glucansucrases (GtfA and Gtf180) derived from *Lactobacillus reuteri.* This reference describes the structural characteristics of glucosylated lactose derivatives synthesized by the enzymes and states that a glucosyl residue could be linked to the reducing glucose unit of lactose (α1→2) by both the enzymes. In addition, Patent document 4 describes an edible composition containing a lactic acid bacterium, an enzyme synthesized by the lactic acid bacterium, and the extracellular polysaccharide (EPS) product of the enzyme. This reference mentions *Lactobacillus* lactic acid bacteria as preferred examples of the lactic acid bacterium, and states that the EPS is obtained after fermentation by a specific species of lactic acid bacteria that can produce a glucansucrase enzyme.

Galactosylkojibiose, which has kojibiose as its constituent sugar, has a structure in which a glucosyl residue is linked to the glucose unit of lactose. With regard to kojibiose and oligosaccharides containing kojibiose as a constituent sugar, Patent document 5 describes an anti-inflammatory agent containing a carbohydrate having an intramolecular ketoaldohexose structure as an active ingredient, and mentions 3-ketotrehalose, 3-ketokojibiose, 3-ketoisomaltose, 3-ketolactose, 3-ketomaltose, 3-ketosucrose, and 3-ketomaltitol as preferred examples of the carbohydrate having an intramolecular ketoaldohexose structure. In addition, Patent document 6 describes an in vivo lipid regulator containing one or more selected from kojibiose, kojitriose, kojibiosyl glucoside, kojitetraose, and kojitriosyl glucoside as an active ingredient.

### Prior Art References

### Patent documents

Patent document 1: Japanese Patent Publication (Kokai) No. Sho 48-98093
Patent document 2: WO2017/091533 (Japanese Patent Publication (Kohyo) No. 2019-505171)
Patent document 3: WO2020/009893
Patent document 4: WO2004/013343 (Japanese Patent Publication (Kohyo) No. 2005-534315)
Patent document 5: WO2017/018336 (Japanese Patent No. 6729913)
Patent document 6: Japanese Patent Publication (Kokai) No. 2005-281188 (Japanese Patent No. 4746281)

### Non-patent documents

Non-patent document 1: Sugars in Sake and Rice Koji Juice. Journal of the Brewing Society of Japan. 53 (11), 859-854, (1958)
Non-patent document 2: Glucan synthesis in the genus Lactobacillus: isolation and characterization of glucansucrase genes, enzymes and glucan products from six different strains - Microbiology (2004), 150, 3681-3990
Non-patent document 3: The production of glucans via glucansucrases from Lactobacillus satsumensis isolated from a fermented beverage starter culture. Gregory L Cote et al., Appl. Microbiol. Biotechnol. (2013) Aug;97(16):7265-73
Non-patent document 4: Structural characterization of glucosylated lactose derivatives synthesized by the Lactobacillus reuteri GtfA and Gtf180 glucansucrase enzymes. Carbohydrate Research. 449 (2017) 59-64

### Summary of the Invention

### Problems to be solved by the invention

When cheese and milk protein concentrate (MPC) are produced from raw milk, whey is produced as a byproduct of the former and membrane permeate is produced as a byproduct of the latter. These mainly contain lactose and minerals, and although they are sometimes used as livestock feed etc., their consumption is limited. In recent years, from the viewpoint of effective utilization of resources, there has been an increasing demand for effective utilization of these byproducts from cheese and milk raw material production. One possible way to utilize these byproducts is to allow a glycosyltransferase to act on lactose in the byproducts to produce oligosaccharides.

As for galactosylkojibiose, which is one of oligosaccharides, and has a structure consisting of lactose and a glucosyl residue linked to the glucose unit of the lactose, it has been found by the studies of the inventors of the present invention to have superior prebiotic properties, and it can be expected as a novel functional ingredient in foods, pharmaceuticals, and cosmetics. However, there are substantially no reports on its production method.

The inventors of the present invention have also found that in the production of galactosylkojibiose, galactosylkojibiose may be isomerized in the process of spray-drying the solution containing this sugar or in the process of heat concentration or heat sterilization of the sugar solution. It is desirable that, even if the process involves heating, at least 80% of galactosylkojibiose should be maintained to be intact.

### Means to solve the problems

The inventors of the present invention mixed a glucansucrase with sucrose and lactose, the sugar acceptor in the glycosyltransfer reaction, and allowed the enzymatic reaction under appropriate conditions. As a result, they found that galactosylkojibiose can be obtained in high yield.

The inventors of the present invention also found that solutions of galactosylkojibiose are more thermally stable on the acidic side, and that the thermal stability tends to decrease when the pH exceeds a specific value. They also identified the chemical structure of the sugar produced by isomerization of galactosylkojibiose.

The present invention provides, in one aspect, the followings.
[1] A method for producing a composition comprising an oligosaccharide, the method comprising:
   allowing a glucansucrase to act on a composition comprising lactose and sucrose to obtain a composition comprising galactosylkojibiose.
[2] The method according to 1, wherein the glucansucrase is used as a lactic acid bacterium or a processed product thereof.
[3] The method according to 2, wherein the lactic acid bacterium is a lactic acid bacterium that constitutively expresses a glucansucrase.
[4] The method according to any one of 1 to 3, wherein the glucansucrase is of bacterial cell-bound type.
[5] The method according to any one of 1 to 4, wherein the glucansucrase has a dextransucrase activity.
[6] The method according to any one of 2 to 5, wherein the lactic acid bacterium or the processed product thereof is any selected from the group consisting of live bacterial cells, dead bacterial cells, culture containing bacterial cells, disrupted bacterial cells, and a purified product of glucansucrase.
[7] The method according to any one of 1 to 6, wherein the glucansucrase, or the lactic acid bacterium that expresses a glucansucrase or the processed product thereof is derived from a lactic acid bacterium belonging to the family *Lactobacillaceae.*
[8] The method according to any one of 1 to 7, wherein the glucansucrase, or the lactic acid bacterium that expresses a glucansucrase or the processed product thereof is derived from a lactic acid bacterium belonging to the genus *Liquorilactobacillus.*
[9] The method according to any one of 1 to 8, wherein the glucansucrase, or the lactic acid bacterium that expresses a glucansucrase or the processed product thereof is derived from a lactic acid bacterium belonging to *Liquorilactobacillus satsumensis.*
[10] A method for producing a composition comprising an oligosaccharide, the method comprising:
   allowing a glucansucrase consisting of the protein (A), (B), or (C) mentioned below to act on a composition comprising lactose and sucrose to obtain a composition comprising galactosylkojibiose:
   (A) a protein consisting of any one of the amino acid sequences of SEQ ID NOS: 1 to 8;
   (B) a protein consisting of an amino acid sequence having a high sequence identity to any one of the amino acid sequences of SEQ ID NOS: 1 to 8 and having a glucansucrase activity; or
   (C) a protein consisting of an amino acid sequence derived from any one of the amino acid sequences of SEQ ID NOS: 1 to 8 by substitution, deletion, insertion, and/or addition of one or multiple amino acids and having a glucansucrase activity.
[11] The method according to 10, wherein the glucansucrase consists of the protein (B') or (C') mentioned below:
   (B') a protein consisting of an amino acid sequence having a high sequence identity to any one of the amino acid sequences of SEQ ID NOS: 1 to 8, in which the portions corresponding to the positions 450 to 467, 488 to 499, and 559 to 573 in the sequence of SEQ ID NO: 1 are identical to those of the sequence of SEQ ID NO: 1, and having a glucansucrase activity; or
   (C') a protein consisting of an amino acid sequence derived from any one of the amino acid sequences of SEQ ID NOS: 1 to 8 by substitution, deletion, insertion, and/or
   addition of one or multiple amino acids, in which the portions corresponding to the positions 450 to 467, 488 to 499, and 559 to 573 in the sequence of SEQ ID NO: 1 are identical to those of the sequence of SEQ ID NO: 1, and having a glucansucrase activity.
[12] A method for producing fermented milk containing galactosylkojibiose, the method comprising:
   allowing a lactic acid bacterium that expresses a glucansucrase or a processed product thereof to act on a composition comprising lactose and sucrose to obtain a composition comprising galactosylkojibiose, and
   adding a lactic acid bacterium starter to raw material milk and allowing fermentation to obtain fermented milk.
[13] An enzyme agent containing a glucansucrase consisting of the protein (A), (B), or (C) mentioned below:
   (A) a protein consisting of any one of the amino acid sequences of SEQ ID NOS: 1 to 8;
   (B) a protein consisting of an amino acid sequence having a high sequence identity to any one of the amino acid sequences of SEQ ID NOS: 1 to 8 and having a glucansucrase activity; or
   (C) a protein consisting of an amino acid sequence derived from any one of the amino acid sequences of SEQ ID NOS: 1 to 8 by substitution, deletion, insertion, and/or addition of one or multiple amino acids and having a glucansucrase activity.
[14] The enzyme agent according to 13, wherein the glucansucrase consists of the protein (B') or (C') mentioned below:
   (B') a protein consisting of an amino acid sequence having a high sequence identity to any one of the amino acid sequences of SEQ ID NOS: 1 to 8, in which the portions corresponding to the positions 450 to 467, 488 to 499, and 559 to 573 in the sequence of SEQ ID NO: 1 are identical to those of the sequence of SEQ ID NO: 1, and having a glucansucrase activity; or
   (C') a protein consisting of an amino acid sequence derived from any one of the amino acid sequences of SEQ ID NOS: 1 to 8 by substitution, deletion, insertion, and/or addition of one or multiple amino acids, in which the portions corresponding to the positions 450 to 467, 488 to 499, and 559 to 573 in the sequence of SEQ ID NO: 1 are identical to those of the sequence of SEQ ID NO: 1, and having a glucansucrase activity.
[15] The enzyme agent according to 13 or 14, wherein the glucansucrase is contained as any selected from the group consisting of live bacterial cells, dead bacterial cells, culture containing bacterial cells, and disrupted bacterial cells of a lactic acid bacterium that expresses a glucansucrase.
[16] The enzyme agent according to any one of 13 to 15, which is used in the method according to any one of 1 to 12.
[17] The enzyme agent according to any one of 13 to 16, which is for producing glucosylepilactose.
[18] A solution containing galactosylkojibiose, which has a pH lower than 7.
[19] A composition comprising galactosylkojibiose, wherein when the composition is made into a solution having a solid content concentration of 10 to 80%, the solution has a pH lower than 7.
[20] The solution according to 18 or the composition according to 19, which contains 10% or more of fructose based on solid content.
[21] The solution according to 18 or the composition according to 19 or 20, which is obtainable by The method according to any one of 1 to 12.
[22] A composition comprising galactosylkojibiose, which has the following characteristics:
   (1) the composition contains 15 to 40% of galactosylkojibiose based on solid content,
   (2) the composition contains 15 to 45% of fructose based on solid content, and
   (3) when the composition is made into a solution having a solid content concentration of 12% and heated at 120°C for 20 minutes, galactosylkojibiose remaining ratio in the solution is 80% or higher.
[23] Glucosylepilactose or a composition comprising glucosylepilactose.
[24] The method according to any one of 1 to 12, which further comprises the step of adjusting pH of the resulting composition to be lower than 7.
[25] The method for producing a composition comprising an oligosaccharide according to any one of 1 to 12 and 24, wherein the composition comprising an oligosaccharide contains galactosylkojibiose and fructose.
[26] The method for producing a composition comprising an oligosaccharide according to any one of 1 to 12, 24 and 25, wherein the composition comprising an oligosaccharide contains 10% or more, but no more than 100% of galactosylkojibiose based on solid content.
[27] The method for producing a composition comprising an oligosaccharide according to any one of 1 to 12 and 24 to 26, wherein the composition comprising an oligosaccharide contains 1% or more of fructose based on solid content.
[28] The method for producing a composition comprising an oligosaccharide according to any one of 1 to 12 and 24 to 27, wherein mass ratio of galactosylkojibiose and lactose (galactosylkojibiose:lactose) in the composition comprising an oligosaccharide is 15 to 40:15 to 30.
[29] The method for producing a composition comprising an oligosaccharide according to any one of 1 to 12 and 24 to 28, wherein the composition comprising an oligosaccharide contains glucosylepilactose.
[30] The method according to any one of 1 to 6 and 24 to 29, which is for producing the solution according to 18 or the composition comprising an oligosaccharide according to any one of 19 to 23.

In one aspect, the present invention provides the followings.
[1] A method for producing a composition comprising an oligosaccharide, the method comprising:
   allowing a lactic acid bacterium that expresses a glucansucrase or a processed product thereof to act on a composition comprising lactose and sucrose to obtain a composition comprising galactosylkojibiose.
[2] The method according to 1, wherein the lactic acid bacterium is a lactic acid bacterium that constitutively expresses the glucansucrase.
[3] The method according to 1 or 2, wherein the glucansucrase is of bacterial cell-bound type.
[4] The method according to any one of 1 to 3, wherein the lactic acid bacterium that expresses a glucansucrase or the processed product thereof is any selected from the group consisting of live bacterial cells, dead bacterial cells, culture containing bacterial cells, disrupted bacterial cells, and a purified product of glucansucrase.
[5] The method according to any one of 1 to 4, wherein the lactic acid bacterium that expresses a glucansucrase or the processed product thereof is derived from a lactic acid bacterium belonging to the family *Lactobacillaceae.*
[6] The method according to any one of 1 to 5, wherein the lactic acid bacterium that expresses a glucansucrase or the processed product thereof is derived from a lactic acid bacterium belonging to the genus *Liquorilactobacillus.*
[7] The method according to any one of 1 to 6, wherein the lactic acid bacterium that expresses a glucansucrase or the processed product thereof is derived from a lactic acid bacterium belonging to *Liquorilactobacillus satsumensis.*
[8] The method according to any one of 1 to 7, which is a method for producing a composition comprising galactosylkojibiose, and comprises the step of purifying galactosylkojibiose from the obtained composition comprising galactosylkojibiose.
[9] A composition comprising galactosylkojibiose, which is obtainable by allowing a lactic acid bacterium that expresses a glucansucrase or a processed product thereof to act on a composition comprising lactose and sucrose.
[10] A method for producing fermented milk containing galactosylkojibiose, the method comprising:
   allowing a lactic acid bacterium that expresses a glucansucrase or a processed product thereof to act on a composition comprising lactose and sucrose to obtain a composition comprising galactosylkojibiose; and
   adding a lactic acid bacterium starter to raw material milk and allowing fermentation to obtain fermented milk.
[11] An enzyme agent containing any selected from the group consisting of live bacterial cells, dead bacterial cells, culture containing bacterial cells, and disrupted bacterial cells of a lactic acid bacterium that expresses a glucansucrase of bacterial cell-bound type.
[12] The enzyme agent according to 11, wherein the lactic acid bacterium is a lactic acid bacterium that constitutively expresses a glucansucrase.
[13] The enzyme agent according to 11 or 12, which is for treating a composition comprising lactose.
[14] Use of a composition comprising lactose, and sucrose as raw materials for obtaining a composition comprising galactosylkojibiose.
[15] The use according to 14, wherein the composition comprising lactose is any selected from the group consisting of raw milk, skimmed milk, reconstituted liquid of dried product containing lactose, skimmed concentrated milk, whey, milk protein concentrate (MPC), whey protein concentrate (WPC), whey protein isolate (WPI), membrane permeate or membrane retained liquid derived from raw milk material, milk concentrate, and lactic acid bacterium culture containing lactose.
[16] The use according to 14 or 15, which is for obtaining galactosylkojibiose with a lactic acid bacterium that expresses a glucansucrase or a processed product thereof.
[17] The use according to 16, wherein the lactic acid bacterium is a lactic acid bacterium that constitutively expresses a glucansucrase.
[18] The use according to 16 or 17, wherein the glucansucrase is of bacterial cell-bound type.

In one aspect, the present invention provides the followings.
[1] A solution containing galactosylkojibiose, which has a pH lower than 7.
[2] The solution according to 1, wherein solid content concentration in the solution is 10 to 80%.
[3] The solution according to 1 or 2 or a dried product thereof, which is obtained by a production method comprising the step of allowing a lactic acid bacterium that expresses a glucansucrase or a processed product thereof to act on a composition comprising lactose and sucrose to obtain a composition comprising galactosylkojibiose.
[4] A composition comprising galactosylkojibiose and glucosylepilactose.
[5] The composition according to 4, which further contains any sugar selected from the group consisting of lactose, fructose, and sucrose, and has a galactosylkojibiose content of 10 to 50% based on solid content.
[6] The composition according to 4 or 5, which is obtainable by a production method comprising the step of allowing a lactic acid bacterium that expresses a glucansucrase or a processed product thereof to act on a composition comprising lactose and sucrose to obtain a composition comprising galactosylkojibiose.
[7] A composition comprising galactosylkojibiose, wherein when the composition is made into a solution having a solid content of 10 to 80%, the solution has a pH lower than 7.
[8] The composition according to 7, which contains 10% or more of fructose based on solid content.
[9] The composition according to 7 or 8, which is obtainable by a production method comprising the step of allowing a lactic acid bacterium that expresses a glucansucrase or a processed product thereof to act on a composition comprising lactose and sucrose to obtain a composition comprising galactosylkojibiose.
[10] A composition comprising galactosylkojibiose, which has the following characteristics:
   (1) the composition contains 15 to 40% of galactosylkojibiose based on solid content,
   (2) the composition contains 15 to 45% of fructose based on solid content, and
   (3) when the composition is made into a solution having a solid content concentration of 12% and heated at 120°C for 20 minutes, galactosylkojibiose remaining ratio in the solution is 80% or higher.
[11] A method for producing a composition comprising an oligosaccharide, the method comprising:
   allowing a lactic acid bacterium that expresses a glucansucrase or a processed product thereof to act on a composition comprising lactose and sucrose to obtain a composition comprising galactosylkojibiose, and
   adjusting pH of the resulting composition to be lower than 7.
[12] Glucosylepilactose or a composition comprising glucosylepilactose.

### Effects of the Invention

Oligosaccharides such as galactosylkojibiose can be efficiently produced from a composition comprising sucrose and lactose as the raw material by the action of glucansucrase according to the production method of one aspect of the present invention.

The production method of one aspect of the present invention enables effective use of byproducts of the production of milk protein concentrate (MPC) etc.

The embodiment of the present invention using a lactic acid bacterium that constitutively expresses a glucansucrase as the lactic acid bacterium that expresses a glucansucrase eliminates the need to add sucrose as an inducer and can prevent byproduction of glucans when preparing a crude enzyme solution.

The embodiment of the present invention using a lactic acid bacterium of bacterial cell-bound type that expresses a glucansucrase as the lactic acid bacterium that expresses a glucansucrase can prevent secretion of glucansucrase into the culture supernatant and thus prevent dilution of the enzyme.

As one aspect of the present invention, there is provided a solution of galactosylkojibiose or a dried product thereof with high stability, especially high thermal stability.

### Brief Description of the Drawings

[Fig. 1] A chromatogram of an enzyme reaction solution obtained by allowing a glucansucrase to act on a mixture of sucrose and lactose.
[Fig. 2] Chromatograms of an enzyme reaction solution, and trisaccharide and tetrasaccharide fractions obtained by fractionation purification.
[Fig. 3] Generation of kojibiose by a lactase treatment of the trisaccharide fraction.
[Fig. 4] Chromatograms of galactosylkojibiose solutions at various pH after a heat treatment.
[Fig. 5] Remaining ratio of galactosylkojibiose after the heat treatment.
[Fig. 6] Analysis of sugars produced by isomerization of galactosylkojibiose.

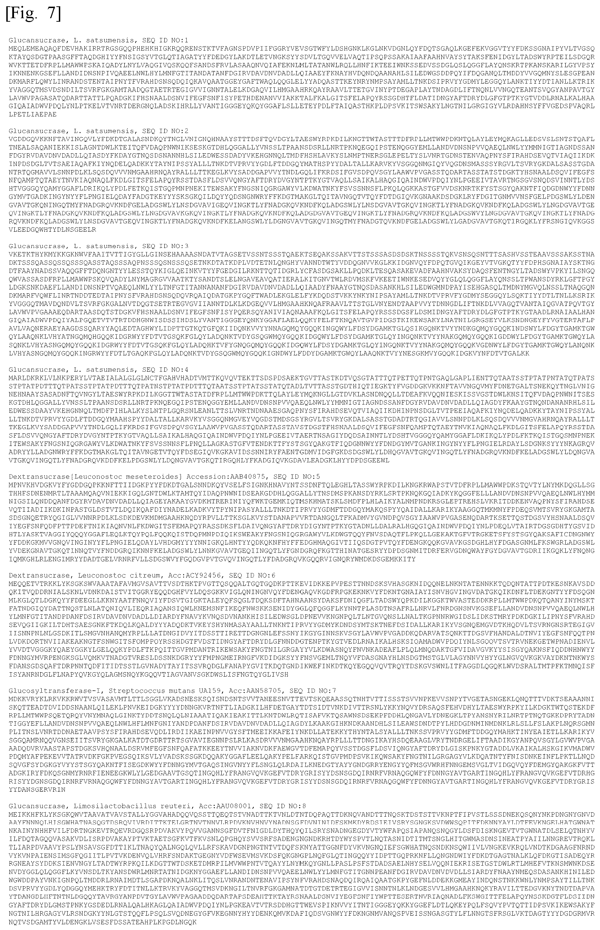

[Fig. 7] Amino acid sequence of a glucansucrase to be used in one embodiment of the present invention. Underlines indicate sites critical for the objective enzyme activity.
[Fig. 8] Amino acid sequence of the glucansucrase to be used in one embodiment of the present invention.

### Modes for Carrying out the Invention

### <Aspect 1: Method for producing composition comprising galactosylkojibiose etc.>

This aspect relates to a method for producing a composition comprising an oligosaccharide such as galactosylkojibiose. This production method comprises the step of allowing a glucansucrase, or a lactic acid bacterium that expresses a glucansucrase or a processed product thereof to act on a composition comprising lactose and sucrose to obtain a composition comprising galactosylkojibiose. The present invention also relates to an enzyme agent containing a lactic acid bacterium that expresses a glucansucrase or a processed product thereof. The present invention further relates to use of byproducts containing lactose obtained in the production process of dairy products.

### [Lactic acid bacterium or processed product thereof]

### (Lactic acid bacterium and glucansucrase)

The production method of this aspect uses a glucansucrase, or a lactic acid bacterium that expresses a glucansucrase or a processed product thereof. For the present invention, glucansucrase refers to an enzyme belonging to the glycoside hydrolase (also called glycosyl hydrolases) family 70 (GH70), unless especially stated (refer to (CAZy) databases, and Cantarel et al., Nucleic Acids Res. 37:D233-238, 2009).

The lactic acid bacterium to be used is not particularly limited so long as it produces the objective glucansucrase. One class of preferred examples consists of lactic acid bacteria belonging to the family *Lactobacillaceae* or *Streptococcus.* When lactic acid bacteria are explained in connection with the present invention, they are explained according to the taxonomy including the reclassification by Zheng J, Wittouck S, Salvetti E, Franz CMAP, Harris HMB, Mattarelli P, O'Toole PW, Pot B, Vandamme P, Walter J, Watanabe K, Wuyts S, Felis GE, Ganzle MG, Lebeer S.: A taxonomic note on the genus Lactobacillus: Description of 23 novel genera, emended description of the genus Lactobacillus Beijerinck 1901, and union of Lactobacillaceae and Leuconostocaceae. Int J Syst Evol Microbiol. 2020 Apr; 70(4): 2782-2858, unless especially indicated.

The family *Lactobacillaceae* includes the following 31 genera:
*Lactobacillus, Paralactobacillus, Holzapfelia, Amylolactobacillus, Bombilactobacillus, Companilactobacillus, Lapidilactobacillus, Agrilactobacillus, Schleiferilactobacillus, Loigolactobacillus, Lacticaseibacillus, Latilactobacillus, Dellaglioa, Liquorilactobacillus, Ligilactobacillus, Lactiplantibacillus, Furfurilactobacillus, Paucilactobacillus, Limosilactobacillus, Fructilactobacillus, Acetilactobacillus, Apilactobacillus, Levilactobacillus, Secundilactobacillus, Lentilactobacillus, Pediococcus, Convivina, Leuconostoc, Fructobacillus, Oenococcus,* and *Weissella.*

In a preferred embodiment, lactic acid bacteria belonging to the family *Lactobacillaceae* excluding lactic acid bacteria belonging to *Limosilactobacillus reuteri* are used, preferably lactic acid bacteria belonging to the family *Lactobacillaceae* excluding lactic acid bacteria belonging to the genus *Limosilactobacillus* are used. In another preferred embodiment, lactic acid bacteria belonging to the genus *Liquorilactobacillus* are used among the lactic acid bacteria belonging to the family *Lactobacillaceae.*

Examples of the lactic acid bacteria belonging to the genus *Liquorilactobacillus* include the followings:
*Liquorilactobacillus aquaticus, Liquorilactobacillus cacaonum, Liquorilactobacillus capillatus, Liquorilactobacillus ghanensis, Liquorilactobacillus hordei, Liquorilactobacillus mali, Liquorilactobacillus nagelii, Liquorilactobacillus oeni, Liquorilactobacillus satsumensis, Liquorilactobacillus sicerae, Liquorilactobacillus sucicola, Liquorilactobacillus uvarum,* and *Liquorilactobacillus vini.*

When a lactic acid bacterium belonging to the family *Streptococcus* is used, it is preferable to use a lactic acid bacterium belonging to the genus *Streptococcus,* and it is more preferable to use a lactic acid bacterium belonging to *Streptococcus mutans.*

In a particularly preferred embodiment, among the lactic acid bacteria belonging to the genus *Liquorilactobacillus,* a lactic acid bacterium that is *Liquorilactobacillus satsumensis* is used. An example of a particularly preferred *Liquorilactobacillus satsumensis* strain is strain JCM12392. *Liquorilactobacillus satsumensis* corresponds to *Lactobacillus satsumensis* according to the taxonomy used before the reclassification. Therefore, the strain JCM12392 is described as *Lactobacillus satsumensis* in RIKEN BioResource Center, GENERAL CATALOG No. 9, 2012, JAPAN COLLECTION OF MICROORGANISMS, M51. The strain JCM12392 is available from RIKEN BioResource Center (RIKEN BRC), Japan Collection of Microorganisms (RIKEN BRC-JCM, Japan) by designating the JCM number JCM12392.

From another viewpoint, it is preferable that the lactic acid bacterium to used be a lactic acid bacterium that constitutively expresses a glucansucrase. The expression of constitutively expressing a glucansucrase means that the gene thereof is expressed without requiring any induction by a substrate or the like. A glucansucrase that is constitutively expressed is referred to as constitutive expression type or constitutive type glucansucrase, while a glucansucrase that requires induction of expression is referred to as an inducible expression type or inducible type glucansucrase. Whether or not a bacterium constitutively expresses a glucansucrase can be confirmed by evaluations of mRNA transcription level by qPCR and protein expression level by Western blotting.

When preparing a lactic acid bacterium that expresses a glucansucrase or a processed product thereof for use in the oligosaccharide production method, sucrose is required for expression of glucansucrase in the case of inducible type glucansucrase. In this case, since sucrose is also a substrate for glucansucrase, it is also involved in the reaction that adds glucose to a glucan chain, and glucans are produced. Glucans having a high molecular weight may make the prepared liquid highly viscous. In the case of constitutive type, sucrose is not required for the preparation of the lactic acid bacterium that expresses glucansucrase or a processed product thereof, and therefore no glucan is produced. The constitutive type is preferred from this point of view.

Examples of the lactic acid bacterium that constitutively expresses a glucansucrase include *Liquorilactobacillus satsumensis.* On the other hand, lactic acid bacteria of the genus *Leuconostoc* are well known as one class of lactic acid bacteria that express glucansucrase, but the glucansucrases expressed by the lactic acid bacteria of the genus *Leuconostoc* are mostly of inducible type.

From another viewpoint, the lactic acid bacterium to be used is preferably one that expresses a glucansucrase of bacterial cell-bound type. It can be determined whether or not the lactic acid bacterium expresses a bacterial cell-bound type glucansucrase by confirming the enzyme activities of the bacterial cells and the culture supernatant, or by confirming whether or not the amino acid sequence of the enzyme or its precursor contains a secretion signal.

When preparing the lactic acid bacterium that expresses a glucansucrase or the processed product thereof for use in the oligosaccharide production method, a bacterial cell-bound type glucansucrase is preferred because such an enzyme can be easily recovered together with the bacterial cells by centrifugation.

The two types of glucansucrases (GtfA and Gtf180) derived from *Lactobacillus reuteri* described in Non-patent document 4 mentioned above are secretory type.

Examples of the glucansucrase to be used in this aspect include a glucansucrase consisting of the protein (A), (B), or (C) mentioned below. The glucansucrase consisting of the protein (A), (B), or (C) mentioned below may be in the form of a lactic acid bacterium that expresses a glucansucrase or a processed product thereof, and in such cases, it can be said that a glucansucrase is used in the production method of this aspect:
(A) a protein consisting of any one of the amino acid sequences of SEQ ID NOS: 1 to 8;
(B) a protein consisting of an amino acid sequence having a high sequence identity to any one of the amino acid sequences of SEQ ID NOS: 1 to 8 and having a glucansucrase activity; or
(C) a protein consisting of an amino acid sequence derived from any one of the amino acid sequences of SEQ ID NOS: 1 to 8 by substitution, deletion, insertion, and/or addition of one or multiple amino acids and having a glucansucrase activity.

In one embodiment, the glucansucrase consists of the protein (B') or (C') mentioned below:
(B') a protein consisting of an amino acid sequence having a high sequence identity to any one of the amino acid sequences of SEQ ID NOS: 1 to 8, in which the portions corresponding to the positions 450 to 467, 488 to 499, and 559 to 573 in the sequence of SEQ ID NO: 1 are identical to those of the sequence of SEQ ID NO: 1, and having a glucansucrase activity; or
(C') a protein consisting of an amino acid sequence derived from any one of the amino acid sequences of SEQ ID NOS: 1 to 8 by substitution, deletion, insertion, and/or addition of one or multiple amino acids, in which the portions corresponding to the positions 450 to 467, 488 to 499, and 559 to 573 in the sequence of SEQ ID NO: 1 are identical to those of the sequence of SEQ ID NO: 1, and having a glucansucrase activity.

The amino acid sequence of GTF21, a constitutively expressed type and bacterial cell-bound type glucansucrase derived from *Liquorilactobacillus satsumensis,* is shown as SEQ ID NO: 1. In this sequence, the portions of the positions 450 to 467, 488 to 499, and 559 to 573 are known to be critical for the glucansucrase activity (Non-patent document 5: J. Agric. Food Chem. 2011, 59, 4148-4155).

The amino acid sequences of glucansucrases of *L. satsumensis* (GTF68, GTF29, and GFT39) having a sequence other than that of SEQ ID NO: 1 are shown as the sequences of SEQ ID NOS: 2 to 4, respectively.

The amino acid sequence of glucansucrase of *Leuconostoc mesenteroides* (accession number AAB40875) is shown as the sequence of SEQ ID NO: 5.

The amino acid sequence of glucansucrase of *Leuconostoc citreum* (accession number ACY92456) is shown as the sequence of SEQ ID NO: 6.

The amino acid sequence of glucansucrase of *Streptococcus mutans* (accession number AAN58705) is shown as the sequence of SEQ ID NO: 7.

The amino acid sequence of glucansucrase of *Limosilactobacillus reuteri* (accession number AAU08001) is shown as the sequence of SEQ ID NO: 8.

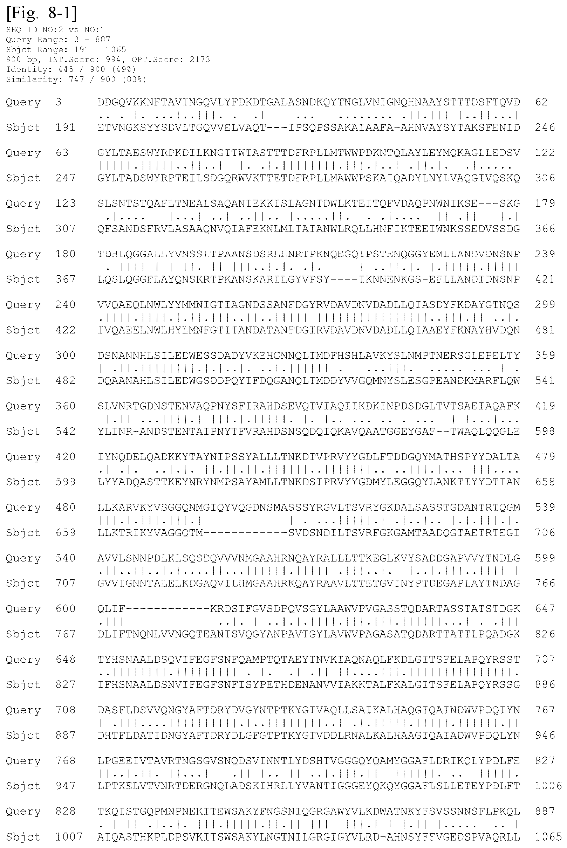

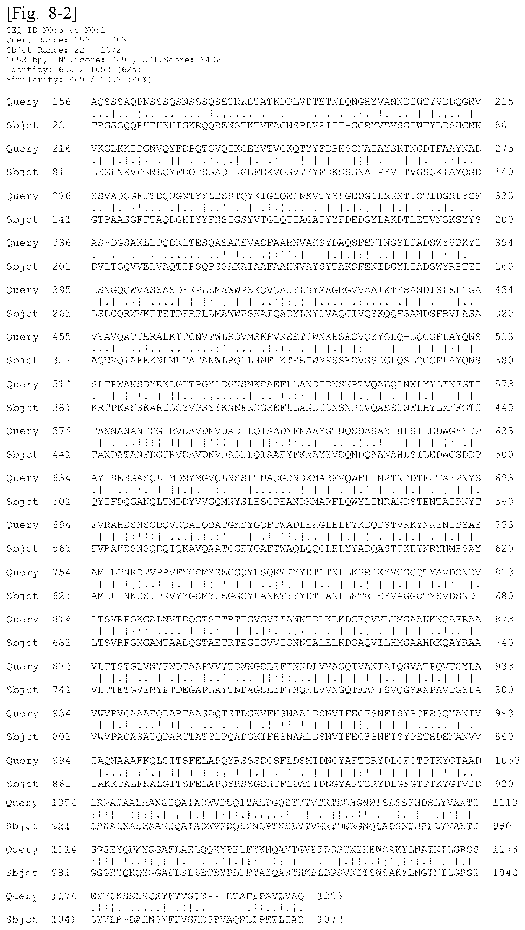

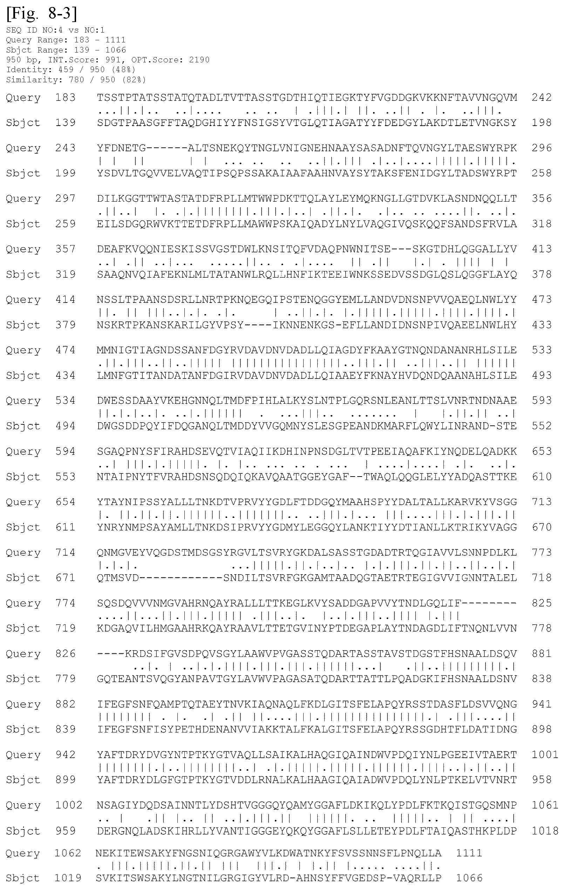

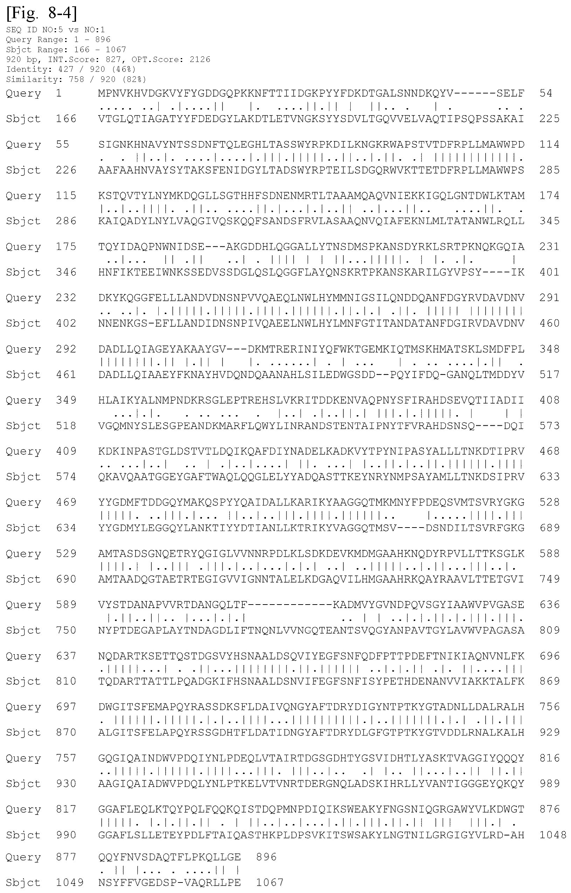

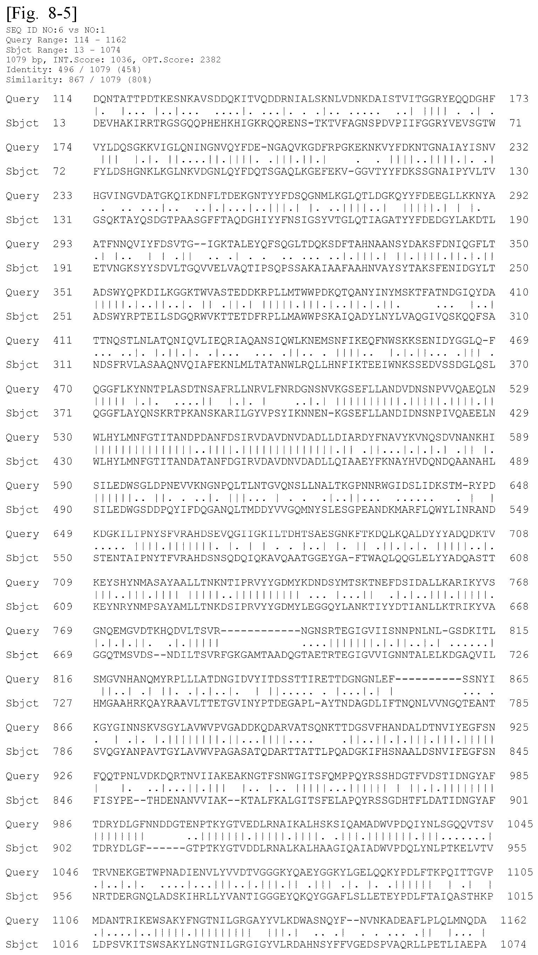

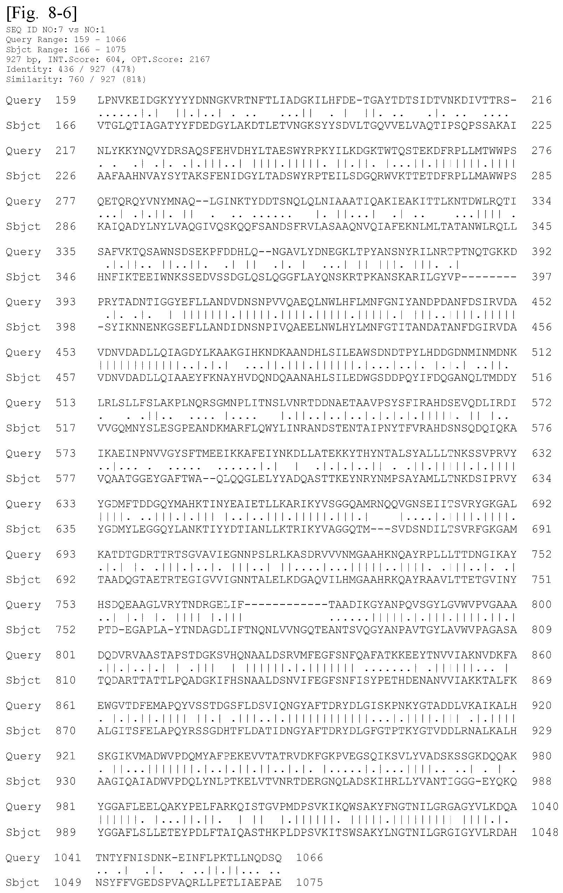

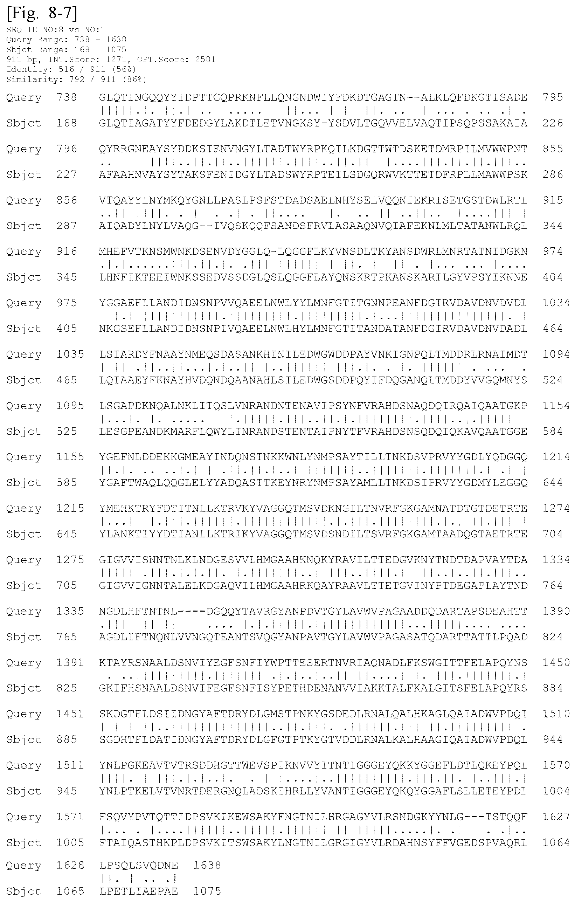

The sequence identities of the sequences to the amino acid sequence of SEQ ID NO: 1 are as shown below and in Fig. 8. These values were specifically calculated by comparing the amino acid sequences of the proteins using the BLAST algorithm (BLASTP, http://www.ncbi.nlm.nih.gov/BLAST/) provided by NCBI (National Center for Biotechnology Information, USA).
SEQ ID NO: 2, 49%
SEQ ID NO: 3, 62%
SEQ ID NO: 4, 48%
SEQ ID NO: 5, 46%
SEQ ID NO: 6, 45%
SEQ ID NO: 7, 47%
SEQ ID NO: 8, 56%

The portion of the positions 559 to 573 in the sequence of SEQ ID NO: 1 corresponds to the conserved sequence 4 described in Non-patent document 5. In one embodiment, the glucansucrase to be used consists of the protein (B) or (C), and one or more, preferably two or more, more preferably three or more, selected from, or further preferably all of:
the amino acid corresponding to the position 565 in the sequence of SEQ ID NO: 1 (H), the amino acid corresponding to the position 566 in the sequence of SEQ ID NO: 1 (D), the amino acid corresponding to the position 567 in the sequence of SEQ ID NO: 1 (S), the amino acid corresponding to the position 571 in the sequence of SEQ ID NO: 1 (D), and
the amino acids corresponding to the position 572 of the sequence of SEQ ID NO: 1 (Q) in the glucansucrase are identical to those of the sequence of SEQ ID NO: 1, regardless of the sequences of the other parts. The portions of the positions 566 and 567 and 571 and 572 of the sequence of SEQ ID NO: 1 are important as the recognition sites for the acceptor (lactose in the case of the present invention) (Non-patent document 5). The amino acids of the positions 565 and 566 of the sequence of SEQ ID NO: 1 are highly conserved among glucansucrases.

The portion of the positions 488 to 499 in the sequence of SEQ ID NO: 1 corresponds to the conserved sequence 3 described in Non-patent document 5. In one embodiment, the glucansucrase to be used consists of the protein (B) or (C), and in this glucansucrase, one or more, preferably two or more, more preferably three or more, selected from, or further preferably all of the amino acids corresponding to the positions 488 to 493 (HLSILE) of the sequence of SEQ ID NO: 1 are identical to those of the sequence of SEQ ID NO: 1, regardless of the sequences of the other parts. In a particularly preferred embodiment, the amino acid corresponding to the position 488 of the sequence of SEQ ID NO: 1 (H) and the amino acid corresponding to the position 493 of SEQ ID NO: 1 (E) are identical to those of the sequence of SEQ ID NO: 1, regardless of the sequences of the other parts.

The portion of the positions 450 to 467 in the sequence of SEQ ID NO: 1 corresponds to the conserved sequence 2 described in Non-patent document 5. In one embodiment, the glucansucrase to be used consists of the protein (B) or (C), and in this case, in the glucansucrase, one or more, preferably two or more, more preferably three or more selected from, or further preferably all of:
the amino acid corresponding to the position 453 in the sequence of SEQ ID NO: 1 (R), the amino acid corresponding to the position 455 in the sequence of SEQ ID NO: 1 (D), the amino acid corresponding to the position 456 in the sequence of SEQ ID NO: 1 (A), and
the amino acids corresponding to the position 458 of the sequence of SEQ ID NO: 1 (D) are identical to those of the sequence of SEQ ID NO: 1, regardless of the sequences of the other parts.

### [Table 1]

The glucansucrase to be used is preferably an enzyme having an activity to catalyze the reaction of degrading sucrose into glucose and fructose and the reaction of adding glucose to a glucan chain, preferably an enzyme having any selected from the dextransucrase (EC 2.4.1.5) activity, alternansucrase (EC 2.4.1.140) activity, reuteransucrase (EC 2.4.1.-) activity, α-4,6-glucanotransferase (EC 2.4.1.-) activity, α-1,2-branched dextransucrase (EC 2.4.1.-) activity, α-4,3-glucanotransferase (EC 2.4.1.-) activity, and mutansucrase (EC 2.4.1.372) activity, more preferably an enzyme having at least the dextransucrase (EC 2.4.1.5) activity.

For the present invention, whether or not a certain protein has the glucansucrase activity can be determined on the basis of whether or not the protein can have the dextransucrase (EC 2.4.1.5) activity, alternansucrase (EC 2.4.1.140) activity, reuteransucrase (EC 2.4.1.-) activity, α-4,6-glucanotransferase (EC 2.4.1.-) activity, α-1,2-branched dextransucrase (EC 2.4.1.-) activity, and α-4,3-glucanotransferase (EC 2.4.1.-) activity. Whether or not a certain protein has the glucansucrase activity is more preferably determined on the basis of whether or not the protein can have the dextransucrase (EC 2.4.1.5) activity.

For the amino acid sequence of glucansucrase, high sequence identity means that the value of identity is 45% or higher, 46% or higher, 47% or higher, 48% or higher, 49% or higher, 50% or higher, 56% or higher, or 62% or higher, preferably 63% or higher, or 70% or higher, more preferably 80% or higher, further preferably 90% or higher, still further preferably 95% or higher, even further preferably 98% or higher.

For the present invention, when identity is referred to for an amino acid sequence, it means percentage of the number of matching amino acids shared between two sequences aligned in an optimal manner, unless especially noted. The search and analysis for amino acid sequence identity can be performed with algorithms or programs well known to those skilled in the art, such as GENETYX (registered trademark) ver. 14 (Genetyx), BLASTN, BLASTP, BLASTX, and ClustalW. When such programs are used, the parameters can be appropriately set by those skilled in the art, or the default parameters of each program can also be used. The specific procedures of these analysis methods are also well known to those skilled in the art.

For the present invention, when referring to an amino acid sequence derived from another amino acid sequence by substitution, deletion, insertion, and/or addition of one or multiple amino acids, the number of the amino acids replaced etc. is not particularly limited as long as the protein consisting of the amino acid sequence has the desired activity, unless especially noted. However, for the glucansucrase consisting of the sequence of SEQ ID NO: 1, of which full length consists of 1075 amino acids, the number is, specifically, for example, 537 or smaller (less than 50% in terms of percentage of the amino acids replaced etc.), preferably 409 or smaller (less than 37% in terms of percentage of the amino acids replaced etc.), more preferably 200 or smaller, further preferably 100 or smaller, further preferably 50 or smaller, further preferably 20 or smaller, further preferably 1 to 9. When the amino acids are replaced with amino acids similar in nature, replacement or the like of a further larger number of amino acids may be possible. Means for preparing polynucleotides for such amino acid sequences or proteins are well known to those skilled in the art.

For the present invention, an amino acid in a certain amino acid sequence S corresponding to the position x (x-th position from the N-terminus) of a reference sequence (e.g., sequence of SEQ ID NO: 1) means an amino acid present at the position of the sequence S that corresponds to the position x of the reference sequence when the sequence S and the reference sequence are aligned. If the sequence S is a sequence derived from the reference sequence by deletion or the like of one or multiple amino acids, the position of the corresponding amino acid in the sequence S may deviate from that in the reference sequence, and may not be the position x in the sequence S. To which amino acid in the sequence S the amino acid at the position x in the reference sequence corresponds can be properly determined by those skilled in the art.

From another point of view, the glucansucrase to be used is preferably one suitable for mass production of an oligosaccharide. Specifically, it is preferably one that can fully exhibit the activity even at high concentration of the substrate. The mass production of an oligosaccharide can be preferably efficiently performed at a higher sucrose concentration from the viewpoint of production efficiency.

From another point of view, the glucansucrase to be used is preferably one of which activity is increased by calcium.

### (Processed product)

The form of the lactic acid bacterium that expresses a glucansucrase or the processed product thereof is not particularly limited as long as they contain a glucansucrase in a state that the glucansucrase has the desired activity. Preferred examples of the lactic acid bacterium that expresses a glucansucrase or the processed product thereof include live bacterial cells, dead bacterial cells, cultures containing bacterial cells, disrupted bacterial cells, and a purified product of a glucansucrase. These may be in a moist or dried state.

Preferred examples of the lactic acid bacterium that expresses a glucansucrase or the processed product thereof to be used in an embodiment wherein a lactic acid bacterium that expresses a glucansucrase of bacterial cell-bound type is used as the lactic acid bacterium that expresses a glucansucrase include live bacterial cells, dead bacterial cells, cultures containing bacterial cells, and disrupted bacterial cells.

Live bacterial cells can be obtained by, for example, culturing a lactic acid bacterium in a commercially available medium, collecting the bacterial cells from the obtained culture medium, and subjecting the cells to such an operation as washing as required. Dead bacterial cell can be obtained by, for example, a sterilization treatment of collected live bacterial cells under conditions that do not inactivate glucansucrase. Culture containing bacterial cells can be obtained by, for example, culturing a lactic acid bacterium in a commercial medium. Disrupted bacterial cells can be obtained by, for example, physically disrupting collected live bacterial cells using a homogenizer, ball mill, bead mill, dyno mill, planetary mill, jet mill, French press, cell crusher, etc., or by disrupting bacterial cell structure by autolysis, enzyme treatment, treatment with a chemical substance such as surfactants, etc.

The degree of purification for the purified product of a glucansucrase is not particularly limited as long as the enzyme can catalyze the desired reaction. The purified product of a glucansucrase may be a highly purified product or a roughly purified product. The purification method is not particularly limited and purification can be performed by conventional techniques. The origin of the purified product of a glucansucrase is not particularly limited as long as it can be used as an enzyme to perform the desired reaction. It may be one purified from cells or culture of lactic acid bacterium, one manufactured as a recombinant protein and purified, or one chemically synthesized and purified.

### (Enzyme agent)

The glucansucrase, or the lactic acid bacterium that expresses a glucansucrase or the processed product thereof can be used as an ingredient of an enzyme agent. When they are used as an ingredient of an enzyme agent, the glucansucrase is preferably a glucansucrase of at least one of the constitutive expression type and the bacterial cell-bound type. It is more preferable that the glucansucrase is of the constitutive expression type and the bacterial cell-bound type. Example of the glucansucrase that is of the constitutive expression type and bacterial cell-bound type include a glucansucrase consisting of the protein (A), (B), or (C) described above.

While the active ingredients of conventional enzyme agents are an enzyme itself, the lactic acid bacterium that expresses a glucansucrase or the processed product thereof as an ingredient of the enzyme agent in one embodiment is not the glucansucrase itself, but may be live bacterial cells, dead bacterial cells, culture containing bacterial cells, or disrupted bacterial cells, as long as they contain the glucansucrase in a state that it has the desired activity. Use of a glucansucrase in such a form as an ingredient of an enzyme agent is particularly suitable when a glucansucrase of the bacterial cell-bound type is used as the ingredient.

In addition to the glucansucrase, or the lactic acid bacterium that expresses a glucansucrase or the processed product thereof, the enzyme agent may contain excipient, buffering agent, suspending agent, stabilizer, preservative, antiseptic, physiological saline etc. Examples of the excipient include starch, dextrin, maltose, trehalose, lactose, D-glucose, sorbitol, D-mannitol, white sugar, and glycerol. Examples of the buffering agent include phosphates, citrates, and acetates. Examples of the stabilizer include propylene glycol and ascorbic acid. Examples of the preservative include phenol, benzalkonium chloride, benzyl alcohol, chlorobutanol, and methylparaben. Examples of the antiseptic include ethanol, benzalkonium chloride, paraoxybenzoic acid, and chlorobutanol.

### (Method for producing glucansucrase)

The glucansucrase can be produced by using a lactic acid bacterium or a processed product thereof, or can also be produced by other methods. For example, the glucansucrase may be produced by expressing it in a suitable expression system. That is, a suitable host can be transformed with a polynucleotide encoding a glucansucrase consisting of the protein (A), (B), or (C) described above, and the glucansucrase can be produced by the resulting transformant. Examples of the host include, for example, bacteria such as *Escherichia coli, Bacillus subtilis,* and lactic acid bacteria, yeasts, and filamentous fungi, and the host can be appropriately selected depending on the type of the recombinant vector, operability, etc. A vector can be used for the transformation, and examples of such a vector include, for example, plasmid vectors, and so forth. The vector can be appropriately selected depending on the type of the host, operability, etc.

The glucansucrase may also be produced by chemical synthesis. That is, the glucansucrase can be synthesized by chemical synthesis methods such as the Fmoc method (fluorenylmethyloxycarbonyl method) and tBoc method (t-butyloxycarbonyl method), or by using various commercial peptide synthesizers on the basis of the amino acid sequence information of the glucansucrase consisting of the protein (A), (B), or (C) described above.

The glucansucrase can be produced by using a genome-edited organism obtained by genome edition of an organism having a gene encoding a glucansucrase. By the genome edition, the expression of the gene encoding the target glucansucrase can be up-regulated. Genome edition techniques that can be used include ZFN, TALEN, CRISPR/Cas9, PPR, and so forth, and those skilled in the art can select an appropriate technique according to the situation.

The enzyme agent can be used in the field of food processing and so forth. Examples include treatments of compositions containing lactose and production of oligosaccharides such as galactosylkojibiose.

### [Composition comprising lactose and sucrose]

The production method of this aspect uses a composition comprising lactose and sucrose.

The composition comprising lactose and sucrose may be one obtained by adding sucrose to a composition comprising lactose. As the composition comprising lactose, byproducts obtained in the production process of dairy products or milk raw materials are preferred, and examples thereof include raw milk, skimmed milk, reconstituted liquid of dried product containing lactose, skimmed concentrated milk, whey, milk protein concentrate (MPC), whey protein concentrate (WPC), whey protein isolate (WPI), membrane permeate (also referred to as permeate) or membrane retained liquid (also referred to as retentate) derived from milk, milk concentrate, and lactic acid bacterium culture containing lactose. The reconstituted liquid of dried product containing lactose means a liquid obtained by reconstituting a known dried product containing lactose derived from milk such as whole milk powder, skimmed milk powder, whey protein concentrate powder, and whey protein isolate powder, and adjusting the resultant liquid to an arbitrary concentration.

Examples of the membrane used in the production of milk raw materials, etc. include microfiltration membranes (MF membranes), ultrafiltration membranes (UF membranes), nanofiltration membranes (NF membranes), and reverse osmosis membranes (RO membranes). Permeate obtained by using an MF or UF membrane among these can be preferably used as the composition comprising lactose in the production method of the present invention. This is because permeate by-produced in the production of a milk raw material such as MPC contains a lot of lactose. When an NF or RO membrane is used, retentate contains lactose.

In a preferred embodiment, a byproduct produced in the production of dairy products or milk raw materials is used as the composition comprising lactose. Examples of such a byproduct include whey produced in the production of cheese, and permeate produced in the production of WPC or MPC. When such permeate is used, the content of acid such as lactic acid is preferably 1% or lower, more preferably 0.8% or lower, still more preferably 0.7% or lower, because pH adjustment for obtaining optimal conditions for the enzymatic reaction is not required at such an acid content.

### [Action of lactic acid bacterium that expresses glucansucrase or processed product thereof]

### (Enzymatic reaction)

In general, glucansucrases have an activity to catalyze the reaction that degrades sucrose into glucose and fructose, and the reaction that adds glucose to a glucan chain. According to the studies of the inventors of the present invention, if lactose, the main ingredient of whey and permeate of membrane concentration of protein, is coexisted as an acceptor in the reaction that a glucansucrase is allowed to act on sucrose, galactosylkojibiose having the kojibiose backbone can be produced. The present invention utilizes such an enzymatic reaction.

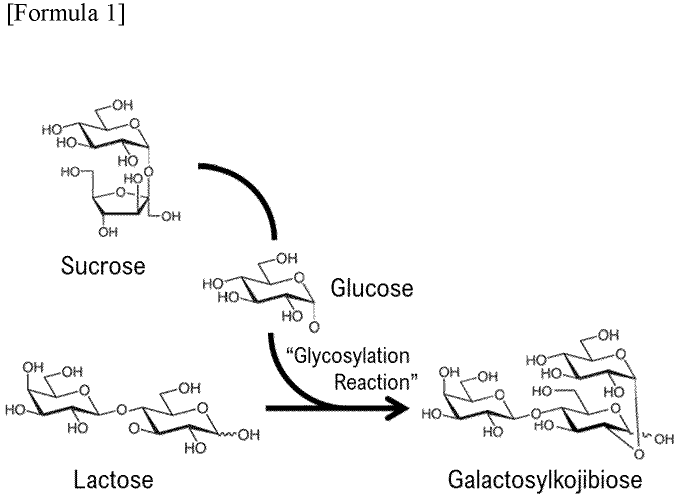

In this reaction, glycosylation occurs with α-1,2 linkage to produce galactosylkojibiose (α-D-glucopyranosyl-(1→2)-[β-D-galactopyranosyl-(1→4)-]D-glucopyranoside). In Non-patent document 3 mentioned above, the structures of high molecular weight glucans produced by a glucansucrase derived from a strain of *Lactobacillus satsumensis* were studied. This reference describes glycosylation with α-1,3 and 1,6 linkages, but does not describe the ability of the enzyme to form α-1,2 linkage by glucosylation.

### (Reaction conditions)

The concentration of lactose at the time of allowing the glucansucrase or the lactic acid bacterium that expresses a glucansucrase or the processed product thereof to act on the composition comprising lactose and sucrose can be determined as appropriate as long as the desired product is obtained. The concentration of lactose can be, for example, 3.0% or higher, and is preferably 6.0% or higher, more preferably 12% or higher, further preferably 15% or higher, still more preferably 20% or higher. On the other hand, it can be 40% or lower, and may be 30% or lower, or 17% or lower.

The concentration of sucrose at the time of allowing the glucansucrase or the lactic acid bacterium that expresses a glucansucrase or the processed product thereof to act on the composition comprising lactose and sucrose can be determined as appropriate as long as the desired product is obtained. The concentration of sucrose can be, for example, 5.0% or higher, and is preferably 10% or higher, more preferably 30% or higher, still more preferably 50%. On the other hand, it can be 64% or lower, and is preferably 60% or lower, more preferably 56% or lower, further preferably 52% or lower. For the present invention, concentrations or ratios of ingredients are indicated as values based on mass, unless especially stated.

The ratio of sucrose and lactose can be selected as appropriate as long as the desired product is obtained. The ratio of sucrose and lactose (sucrose:lactose) can be, for example, 0.5:1 to 11:1, and is preferably 0.7:1 to 9:1, more preferably 0.8:1 to 6:1, further preferably 1:1 to 4:1.

The concentration of the lactic acid bacterium that expresses a glucansucrase or the processed product thereof at the time of allowing the lactic acid bacterium that expresses a glucansucrase or the processed product thereof to act on the composition comprising lactose and sucrose can be determined as appropriate as long as the desired product is obtained. When the lactic acid bacterium that expresses a glucansucrase is used as live bacterial cells, the lactic acid bacterium can be cultured under appropriate conditions, and a suspension in which the bacterial cells collected from the resulting culture medium, and washed, if necessary, are suspended in a buffer solution in a volume of 0.2 to 2 times the volume of the original culture medium can be used as the lactic acid bacterium that expresses a glucansucrase or the processed product thereof referred to here. Such a suspension of the lactic acid bacterium can be added to the system in an amount of 1.0 to 25%, preferably 2.0 to 20%, more preferably 3.0 to 18%, further preferably 4.0 to 15%, of the system. Alternatively, a lactic acid bacterium can be cultured under appropriate conditions, and the concentrate of the culture medium can be used as the lactic acid bacterium that expresses a glucansucrase or the processed product thereof referred to here. Such a concentrate of lactic acid bacterium can be added to the system in an amount of 0.01 to 10%, preferably 0.05 to 5%, 0.1 to 3%, or 0.2 to 0.6% of the system.

The system in which the lactic acid bacterium that expresses a glucansucrase or the processed product thereof is allowed to act on the composition comprising lactose and sucrose may contain an ingredient that increases the activity of the glucansucrase, and preferably does not contain any ingredient that inhibits the activity. When the glucansucrase is a glucansucrase consisting of the protein (A), (B), or (C) mentioned above, examples of the ingredient that increases the activity of the glucansucrase include calcium, and examples of the ingredient that inhibits the activity include iron ion.

The conditions such as temperature for allowing the lactic acid bacterium that expresses a glucansucrase or the processed product thereof to act on the composition comprising lactose and sucrose can be appropriately set by those skilled in the art.

The pH can be 3.5 to 7.0, and is preferably 4.0 to 6.5, more preferably 4.5 to 6.0.

The temperature can be 4 to 55°C, and is preferably 20 to 52°C, more preferably 30 to 51°C, further preferably 46 to 50°C.

The time of allowing the lactic acid bacterium that expresses a glucansucrase or the processed product thereof to act on the composition comprising lactose and sucrose can be set so that the desired purified product can be sufficiently obtained. The time for that is usually 4 to 96 hours, preferably 6 to 80 hours, more preferably 8 to 72 hours, further preferably 12 to 64 hours.

### [Composition comprising galactosylkojibiose and final product]

The production method of the present invention comprises the step of allowing a glucansucrase, or a lactic acid bacterium that expresses a glucansucrase or a processed product thereof to act on a composition comprising lactose and sucrose to obtain a composition comprising galactosylkojibiose.

The production method of the present invention can be carried out for the purpose of producing a composition comprising various oligosaccharides. The objective final product, oligosaccharide, is, for example, any selected from the group consisting of kojibiose and oligosaccharides containing kojibiose as a constituent sugar. For the present invention, the term "oligosaccharide containing kojibiose as a constituent sugar" does not include kojibiose itself. For the present invention, "any" is used to mean "at least one", unless especially stated.

Examples of the oligosaccharide containing kojibiose as a constituent sugar include galactosylkojibiose, which is a trisaccharide, and tetrasaccharides consisting of galactosylkojibiose linked with one molecule of monosaccharide such as glucose. For the present invention, galactosylkojibiose refers to kojibiose mentioned below α- or β-linked with galactose at any of the positions 1 to 8 of kojibiose, unless especially stated. Theoretically, galactose can be linked to the OH at the position 8 in four ways (α-α, α-β, β-α, and β-β) and to the OH at the positions 2 to 7 in two ways, and so there are 18 possible types of galactosylkojibiose.

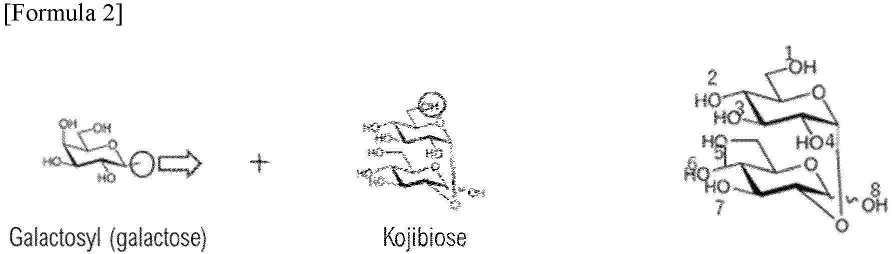

In a preferred embodiment, one type of galactosylkojibiose to be produced is α-D-glucopyranosyl-(1→2)-[β-D-galactopyranosyl-(1→4)-]D-glucopyranoside). This sugar can also be described as one type of glucosyllactose.

For the present invention, glucosyllactose refers to glucose linked to lactose, unless especially stated. More specifically, it refers to any selected from the compounds consisting of lactose and glucose α- or β-linked at any of the positions 1 to 8 of the lactose described below. Theoretically, glucose can be linked to the OH at the position 1 in four ways (α-α, α-β, β-α, and β-β) and to the OH at the positions 2 to 7 in two ways, and so there can be possible 18 types of glucosyllactose. They are called 1α-α, 1α-β, 1β-α, 1β-β, 2α, 2β, 3α, 3β, 4α, 4β, 5α, 5β, 6α, 6β, 7α, 7β, 8α, and 8β isomers, respectively. α-D-Glucopyranosyl-(1→2)-[β-D-galactopyranosyl-(1→4)-]D-glucopyranoside consists of lactose and glucose α-linked to the OH at the position 8 of the lactose (8α isomer).

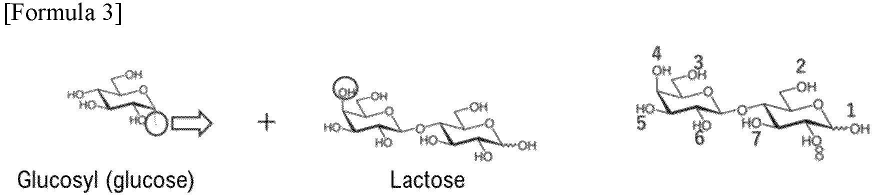

In one embodiment, glucosyllactose other than α-D-glucopyranosyl-(1→2)-[β-D-galactopyranosyl-(1-4)-]D-glucopyranoside can be produced whether or not the above-mentioned 8α isomer is produced. More precisely, any one of the 17 types of glucosyllactose other than the 8α isomer (1α-α, 1α-β, 1β-α, 1β-β, 2α, 2β, 3α, 3β, 4α, 4β, 5α, 5β, 6α, 6β, 7α, 7β, and 8β isomers) may be produced, and 2, 3, 4, 5, 6, 7, 8, 9, 10, 11, 12, 13, 14, 15, 16, or 17 types of glucosyllactose may be produced.

### (Sugars contained in composition)

The composition obtained by the step of allowing a glucansucrase, or a lactic acid bacterium that expresses a glucansucrase or a processed product thereof to act on a composition comprising lactose and sucrose may contain unreacted lactose in addition to galactosylkojibiose, and may also contain fructose produced by the decomposition of sucrose. Fructose contained in the obtained composition is usually contained in an equimolar amount or larger amount relative to galactosylkojibiose. The composition may also contain a tetrasaccharide consisting of galactosylkojibiose, which is a trisaccharide, and one molecule of a monosaccharide such as glucose linked to the galactosylkojibiose.

In the explanations of the present invention and the embodiments thereof, the production method of the present invention may be explained by referring to the case where the composition comprising an oligosaccharide as the final product is a composition comprising galactosylkojibiose, but such explanations can also be applied to the case where a composition comprising a tetrasaccharide consisting of galactosylkojibiose and one molecule of a monosaccharide such as glucose linked to the galactosylkojibiose. Those skilled in the art can understand such explanations as mentioned above by replacing the term galactosylkojibiose with tetrasaccharide consisting of galactosylkojibiose and one molecule of a monosaccharide such as glucose linked to the galactosylkojibiose.

### (Form and others)

The composition obtained by the production method can be in various forms. The composition may the obtained reaction solution as it is, or may be subjected to the purification step described below, if necessary, and then made into concentrate, frozen product, or dried product. The dried product can be in the form of powder or granule.

### (Use)

The obtained composition can be used in the fields of foods, cosmetics, and pharmaceuticals as sweetener, taste improver, quality improver, stabilizer, anti-discoloration agent, excipient, or such a functional ingredient as described below. The foods and pharmaceuticals include not only those for humans, but also those for animals other than humans, unless especially stated. The foods include general foods, functional foods, and nutritional compositions, therapeutic foods (foods that can achieve therapeutic purposes; foods prepared on the basis of a menu prepared by a nutritionist, etc. in accordance with a diet slip prescribed by a medical practitioner), dietetic foods, foods with adjusted ingredients, nursing care foods, and foods for therapeutic support, unless especially stated. The foods also include not only solids, but also liquids, e.g., beverages, drinkable preparations, and soups, unless especially noted.

The composition can also be used as a functional ingredient. According to the studies of the inventors of the present invention, kojibiose and oligosaccharides containing kojibiose as a constituent sugar, such as galactosylkojibiose, can promote the growth of *Parabacteroides* bacteria in the intestinal microbiota and the growth of *Bifidobacterium* bacteria in the intestinal microbiota. Therefore, the composition obtained by the production method of the present invention can be used to promote the growth of *Parabacteroides* bacteria in the intestinal microbiota, and to promote the growth of *Bifidobacterium* bacteria in the intestinal microbiota. In addition, since it can be expected that inflammation, obesity, abnormal glucose metabolism, multiple sclerosis (MS), hepatic lipidosis, cancer, and chronic obstructive pulmonary disease (COPD) can be treated by the promotion of the growth of *Parabacteroides* bacteria, the composition can be used for the treatment of these diseases and for the treatment of diseases or conditions other than those listed above that can be improved by the promotion of the growth of *Parabacteroides* bacteria in the intestine. Furthermore, since promotion of mineral absorption and intestinal regulation by the promotion of the growth of *Bifidobacterium* bacteria in the intestinal microbiota can be expected, the composition can be used for promotion of mineral absorption, intestinal regulation, and treatment of conditions other than those listed above that can be improved by promotion of the growth of *Bifidobacterium* bacteria in the intestines.

The composition can also be used as a prebiotics or synbiotics.

### [Specific embodiments]

### (Other steps)

The production method of the present invention may further comprise various steps in addition to the step of allowing a glucansucrase, or a lactic acid bacterium that expresses a glucansucrase or a processed product thereof to act on a composition comprising lactose and sucrose to obtain a composition comprising galactosylkojibiose.

The production method may further comprise a purification step. Examples of the target ingredient of the purification in the purification step include galactosylkojibiose and a tetrasaccharide consisting of galactosylkojibiose and a single molecule of monosaccharide such as glucose linked to the galactosylkojibiose.

The purification step may comprise, for example, any of such means as a treatment with activated carbon, separation by chromatography, treatment with ion exchange resin, filtration, concentration, crystallization, crystal separation, drying, cooling, temperature and humidity control.

When a purification step is performed, degree of the purification is not particularly limited. For example, the purity of galactosylkojibiose can be 30% or higher, and may be 40% or higher, 50% or higher, 60% or higher, 70% or higher, 80% or higher, 90% or higher, 95% or higher, or 97% or higher.

The production method may further comprise a pH adjustment step. The agent for pH adjustment used in the pH adjustment step is not particularly limited, so long as an agent suitable for food, pharmaceutical, and cosmetic productions is selected. Examples include organic acids such as citric acid, malic acid, gluconic acid, succinic acid, ascorbic acid, tartaric acid, lactic acid, and fumaric acid, inorganic acids such as phosphoric acid, and salts thereof. Adjustment of the resulting composition to an appropriate pH may be important for stabilization of objective ingredients contained.

The pH can be adjusted to, for example, 3.0 to 8.0, and pH may be 4.0 to 7.0, or 4.5 to 6.0.

The production method preferably further comprises a sterilization step. The stage at which the sterilization step is carried out is not particularly limited, and if necessary, multiple sterilization steps may be carried out. For example, the composition comprising lactose and sucrose can be sterilized prior to allowing the glucansucrase, or the lactic acid bacterium that expresses a glucansucrase or the processed product thereof to act on the composition comprising lactose and sucrose. Alternatively, after the glucansucrase, or the lactic acid bacterium that expresses glucansucrase or the processed product thereof is allowed to act on the composition comprising lactose and sucrose, the resulting composition comprising galactosylkojibiose can be sterilized under conditions that the glucansucrase is inactivated. If the obtained composition comprising galactosylkojibiose is to be concentrated, the composition may be sterilized before the concentration step.

The means for the sterilization is not particularly limited as long as a mean that does not affect the objective ingredient is used. Examples include heat sterilization (UHT sterilization, retort sterilization, etc.) and ultraviolet sterilization.

### (Exemplary indications)

As one embodiment, there can be implemented a method for producing fermented milk containing galactosylkojibiose, which comprises the following steps:
the enzyme treatment step of allowing a lactic acid bacterium that expresses a glucansucrase or a processed product thereof to act on a composition comprising raw material milk and sucrose to obtain a composition comprising galactosylkojibiose; and
the fermentation step of adding a lactic acid bacterium starter to raw material milk and allowing fermentation to obtain fermented milk.

The order of the enzyme treatment step and the fermentation step is not particularly limited. For example, after the enzyme treatment step is performed, a lactic acid bacterium starter may be added, and the fermentation step may be performed, or after the fermentation step is performed, a lactic acid bacterium that expresses a glucansucrase or a processed product thereof may be added to perform the enzyme treatment step. These steps allow the fermented milk to contain galactosylkojibiose without external addition thereof.

Examples of the raw material milk include raw milk, cow's milk, special milk, skimmed milk and partially skimmed milk, as well as mixtures of any of these.

The lactic acid bacterium used as the starter is not particularly limited as long as a lactic acid bacterium suitable for the production of fermented milk is chosen. It is preferable to use *Lactobacillus delbrueckii.* As *Lactobacillus delbrueckii,* it is preferable to used one classified as *Lactobacillus delbrueckii* subsp. *bulgaricus. Streptococcus thermophilus* may also be used. *Lactobacillus delbrueckii* and *Streptococcus thermophilus* may be used in combination.

As an embodiment, there can be implemented a method for producing a milk concentrate containing galactosylkojibiose, which comprises the following steps:
the step of allowing a glucansucrase, or a lactic acid bacterium that expresses a glucansucrase or a processed product thereof to act on raw material skimmed concentrated milk to obtain skimmed concentrated milk containing galactosylkojibiose; and
the step of drying the resulting skimmed concentrated milk, as desired.

Examples of the milk concentrate obtainable in this embodiment include, for example, concentrated milk (product obtained by concentrating raw milk, cow's milk, or special milk, with a milk solid content of 25.5% or higher and a milk fat content of 7.0% or higher), and skimmed concentrated milk (product obtained by concentrating raw milk, cow's milk, or special milk from which milk fat had been removed, with a nonfat milk solid content of 18.5% or higher, and a bacterial count (per 1 gram as determined by the standard plate culture method) of 100,000 or lower, as defined in the "Ordinance of the Ministry of Health and Welfare Concerning Standards of Ingredients of Milk and Milk Products", as well as a product obtained by concentrating raw milk, cow's milk, or special milk, with a milk solid content lower than 25.5% (concentrated milk other than the concentrated milk specified by ministerial ordinance), a product obtained by concentrating raw milk, cow's milk, or special milk from which milk fat had been removed, with a nonfat milk solid content lower than 18.5% (skimmed concentrated milk other than skimmed concentrated milk specified by ministerial ordinance), and milk protein concentrate (MPC).

### <Aspect 2: Composition comprising galactosylkojibiose etc.>

This aspect of the present invention relates to a solution containing galactosylkojibiose, which has a pH less than or equal to a specific value, and a composition comprising galactosylkojibiose and glucosylepilactose.

### [Composition]

### (Galactosylkojibiose, pH of solution, and content)

The composition of this aspect contains galactosylkojibiose (already defined in this description).

In a preferred embodiment, the composition contains one type of galactosylkojibiose, α-D-glucopyranosyl-(1→2)-[β-D-galactopyranosyl-(1→4)-]D-glucopyranoside).

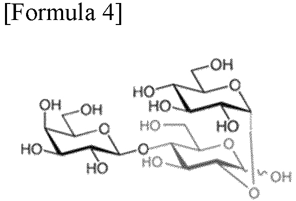

This α-D-glucopyranosyl-(1→2)-[β-D-galactopyranosyl-(1→4)-]D-glucopyranoside is also referred to as 4-galactosylkojibiose or 4'-galactosylkojibiose. It is also sometimes referred to as one type of glucosyllactose (already defined in this description).

The composition may contain, in one embodiment, glucosyllactose other than α-D-glucopyranosyl-(1→2)-[β-D-galactopyranosyl-(1→4)-]D-glucopyranoside whether or not the composition contains that sugar. Precisely, any one of 17 types of glucosyllactose other than the above sugar (1α-α, 1α-β, 1β-α, 1β-β, 2α, 2β, 3α, 3β, 4α, 4β, 5α, 5β, 6α, 6β, 7α, 7β, and 8β isomers) may be produced, and the composition may contain 2, 3, 4, 5, 6, 7, 8, 9, 10, 11, 12, 13, 14, 15, 16, or 17 types of these.

The content of galactosylkojibiose in the composition is not particularly limited. For example, it can be 10% or higher, or 15% or higher, and is preferably 20% or higher, more preferably 25% or higher, further preferably 30% or higher, based on solid content. The maximum content is not particularly limited, but the content may be, for example, 100%, 99% or lower, 80% or lower, 70% or lower, 50% or lower, or 40% or lower. In one of the preferred embodiments, the content of galactosylkojibiose in the composition can be 10 to 50%, and is more preferably 15 to 40%, further preferably 30 to 40%, based on solid content. For the present invention, contents or concentrations of ingredients numerically indicated are values based on mass, unless especially stated.

In one of the preferred embodiments, the composition is a solution (sugar solution) having a pH lower than 7, or a composition of which solution having a solid content concentration of 10 to 80% has a pH lower than 7. This is because a pH lower than 7 provides high thermal stability of galactosylkojibiose. The pH can be, for example, 2 or higher, 2.5 or higher, or 3 or higher, and is preferably not lower than 3 and not higher than 6, more preferably not lower than 4 and not higher than 6. This is because such pH range provides higher thermal stability of galactosylkojibiose. The pH of the solution can be measured, if appropriate, at a solid content concentration in the solution of 12%. For the present invention, pH can be measured by the usual measurement methods used in this field.

In one of the preferred embodiments, the composition is a concentrated, frozen, or dried product containing galactosylkojibiose of which solution having a solid content concentration of 10 to 80% (aqueous solution) has a pH lower than 7. The dried product is a product obtained by drying the solution to a moisture content of 7% or lower, preferably 5% or lower. The moisture content can be measured by the official method for measuring moisture content of sugars used in the field of food or defined in the Japanese Agricultural Standards (JAS). Examples of the dried product include powders and granules. The pH of the solution of the concentrated, frozen, or dried product with a solid content of 10 to 80% may be, for example, 2 or higher, 2.5 or higher, or 3 or higher, and is preferably not lower than 3 and not higher than 6, more preferably not lower than 4 and not higher than 6. This is because the thermal stability of galactosylkojibiose is higher in such ranges. The pH of the solution can be measured, if appropriate, at a solid content concentration in the solution of 12%.

For the present invention, the solid content concentration refers to the concentration of solids dissolved in the solution. The solid content concentration can be measured by the methods usually used in this field. The solid content concentration can be expressed as a Brix value or sugar content, if appropriate, and Brix value or sugar content can be generally measured by using known measurement methods as appropriate, and can generally be measured by using commercially available instruments (e.g., digital refractometer of the trade name "RX-5000α" (Atago)).

High thermal stability means that when a solution of galactosylkojibiose having a solid content concentration of 12% is heated at 120°C for 20 minutes, the remaining ratio of galactosylkojibiose is 80% or higher, preferably 90% or higher, more preferably 95% or higher.

When the composition comprising galactosylkojibiose is a solution, the solid content concentration of the solution is not particularly limited, but is, for example, 10% or higher, preferably 30% or higher, more preferably 50% or higher, further preferably 60% or higher. The maximum solid content concentration is not particularly limited, but the solid content concentration can be, for example, 80% or lower, and may be 78% or lower, or 76% or lower. The solid content mainly consists of sugars, but may contain ingredients other than sugars. Examples of such ingredients other than sugars include the enzyme used in the production method described below and agent for pH adjustment. Influences of such ingredients may not be significant, and may be negligible.

### (Other ingredients and chemical composition)

The composition may contain, in addition to galactosylkojibiose, glucosylepilactose, more specifically glucosylepilactose (α-D-Glcp-(1→2)-[β-D-Galp-(1-4)-]D-Manp). This sugar may also be referred to as 2-glucosylepilactose. In the explanations of the present invention and embodiments thereof, the term glucosylepilactose refers to a trisaccharide having the following structure, unless especially stated.

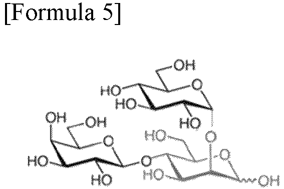

The composition may further contain α-D-glucopyranosyl-(1→2)-[β-D-galactopyranosyl-(1-4)-]D-glucopyranoside mentioned above, and may contain a glucosyllactose other than the above sugar whether or not the composition contains the above sugar.

Glucosylepilactose is a novel compound of which production in a heat-treated galactosylkojibiose solution was found by the inventors of the present invention.

Glucosylepilactose can be produced by adjusting a composition comprising galactosylkojibiose to pH 7 or higher and then heating it to 100°C or higher, preferably 110°C or higher.

When the composition contains glucosylepilactose, the content of glucosylepilactose is not particularly limited, but the composition may contain, for example, 5% or less, preferably 3% or less, more preferably 1% or less, further preferably 0.1% or less, of glucosylepilactose based on solid content. The minimum content is not particularly limited, but the composition may contain, for example, 0.001% or more, 0.01% or more, or 0.05% or more of glucosylepilactose based on solid content.

When the composition contains glucosylepilactose, the mass ratio of galactosylkojibiose and glucosylepilactose (galactosylkojibiose:glucosylepilactose) is not particularly limited, but it is, for example, 99.99:0.01 to 80:20, and may be 99.9:0.1 to 85:15, or 99:1 to 90:10.

The composition may also further contain any sugar selected from the group consisting of lactose, fructose, and sucrose. For the present invention, the term "any" is used to mean "at least one", unless especially stated.

If the composition contains fructose, content of fructose is not particularly limited, but the composition may contain, for example, 5% or more, preferably 15% or more, more preferably 20% or more, further preferably 30% or more, of fructose based on solid content. The maximum content is not particularly limited, but the content is, for example, 45% or lower, 40% or lower, or 35% or lower based on solid content. If a high purity of galactosylkojibiose is preferred, a lower fructose content is preferred, and it is more preferred that fructose is not contained.

If the composition contains fructose, the mass ratio of galactosylkojibiose and fructose (galactosylkojibiose: fructose) is not particularly limited, but it is, for example, 15 to 40:15 to 45, and may be 17 to 37:17 to 37, or 20 to 35:25 to 40.

If the composition contains lactose, content of lactose is not particularly limited, but the composition may contain, for example, 30% or less, preferably 25% or less, more preferably 20% or less, further preferably 15% or less of lactose based on solid content. The minimum content is not particularly limited, but the content is, for example, 1% or higher, 5% or higher, or 10% or higher based on solid content. If a high purity of galactosylkojibiose is preferred, a lower lactose content is preferred, and it is more preferred that lactose is not contained..

If the composition contains lactose, the mass ratio of galactosylkojibiose and lactose (galactosylkojibiose:lactose) is not particularly limited, but it is, for example, 15 to 40:15 to 30, and may be 17 to 37:17 to 37, or 20 to 35:20 to 25.

When the composition contains sucrose, content of sucrose is not particularly limited, but the composition may contain, for example, 30% or less, preferably 25% or less, more preferably 20% or less, further preferably 15% or less, 10% or less, 5% or less, 1% or less, 0.5% or less, 0.2% or less, or 0.1% or less of sucrose based on solid content. The minimum content is not particularly limited, but the content may be, for example, 0.01% or higher, 0.5% or higher, 1% or higher, 5% or higher, or 10% or higher based on solid content. All sucrose may be utilized in the reaction depending the conditions and the content may be below the detection limit.

If the composition contains sucrose, the mass ratio of galactosylkojibiose and sucrose (galactosylkojibiose:sucrose) is not particularly limited, but it is, for example, 15 to 40:15 to 30, and may be 17 to 37:17 to 37, or 20 to 35:20 to 25.

The composition may also contain a tetrasaccharide consisting of galactosylkojibiose, a trisaccharide, and one molecule of a monosaccharide such as glucose linked to the galactosylkojibiose.

One preferred embodiment is a composition comprising galactosylkojibiose, which has the following characteristics
(1) the composition contains 15 to 40% of galactosylkojibiose based on solid content,
(2) the composition contains 15 to 45% of fructose based on solid content, and
(3) when the composition is made into a solution having a solid content concentration of 12% and heated at 120°C for 20 minutes, galactosylkojibiose remaining ratio in the solution is 80% or higher.

The above composition may further have the following characteristic:
(4) the composition contains 5 to 30% of lactose based on solid content.

The above composition may have the following characteristic instead of or in addition to the characteristic (3):
(6) when the composition is made into a solution having a solid content concentration of 10 to 80%, the solution has a pH lower than 7.

Such a composition is highly stable and suitable for processing. It can also be expected to be suitable for long-term storage and distribution. In addition, it may be useful because of its superior taste quality, lower calorie than sucrose in the same amount etc.

### (Form)

The composition can be in various forms. In one preferred embodiment, the composition is a solution. In one embodiment, the form of the composition may be a solution obtained by the production method described below, i.e., the production method comprising the step of allowing a glucansucrase, or a lactic acid bacterium that expresses glucansucrase or a processed product thereof to act on a composition comprising lactose and sucrose to obtain a composition comprising galactosylkojibiose (also referred to as "reaction product").

The solution can be made into a concentrated, frozen, or dried product. The dried product is a product obtained by drying the solution to a moisture content of 7% or lower, preferably 5% or lower. The moisture content can be measured by the official method for measuring moisture content of sugars used in the field of foods or defined in the Japanese Agricultural Standards (JAS). Examples of the dried product include powders and granules.

### (Use)

The composition comprising galactosylkojibiose can be used in the fields of foods, cosmetics, and pharmaceuticals as sweetener, taste improver, quality improver, stabilizer, anti-discoloration agent, excipient, or functional ingredient described below. The descriptions in the section of (Use) of the above <Method for producing composition comprising galactosylkojibiose etc.> can be applied to the composition of this aspect as they are.

Glucosylepilactose that may be contained in one embodiment of the composition is expected to be useful as a precursor of epilactose, which has been reported to be a promising prebiotics (Non-patent document 5 mentioned below). Therefore, the composition is used to obtain glucosylepilactose as well as a precursor of epilactose.

### [Method for producing composition]

The composition comprising galactosylkojibiose of this aspect can be produced by various methods. One particularly preferred example of the production method is the production method comprising the step of allowing a glucansucrase, or a lactic acid bacterium that expresses a glucansucrase or a processed product thereof to act on a composition comprising lactose and sucrose to obtain a composition comprising galactosylkojibiose. The descriptions of <Aspect 1: Method for producing composition comprising galactosylkojibiose etc.> mentioned above can be applied to the production of the composition of this aspect as they are.

### (pH Adjustment step)

The method for producing the composition of this aspect may further comprise a pH adjustment step. Although pH adjustment is not required when pH is lower than 7, a step of confirming the pH may also be regarded as the pH adjustment step. The agent for pH adjustment used in the pH adjustment step is not particularly limited as long as an agent suitable for food, pharmaceutical or cosmetic production is used. Examples include organic acids such as citric acid, malic acid, gluconic acid, succinic acid, ascorbic acid, tartaric acid, lactic acid, and fumaric acid, inorganic acids such as phosphoric acid, and salts thereof. Adjustment of the resulting composition to an appropriate pH may be important for stabilization of the objective ingredients contained.

The pH can be adjusted to be, for example, lower than 7, because a pH lower than 7 results in higher thermal stability of galactosylkojibiose. The pH may be, for example, 2 or higher, 2.5 or higher, or 3 or higher, and is preferably not lower than 3 and not higher than 6, more preferably not lower than 4 and not higher than 6. This is because the thermal stability of galactosylkojibiose is higher in this pH range. The pH of the solution can be measured, if appropriate, at a solid content concentration of 12% of the solution.

### Examples

### [Example 1: Production of galactosylkojibiose using lactose]

### (Preparation of enzyme solution)

*Lactobacillus satsumensis* JCM12392 was cultured overnight at 30°C in a commercial MRS liquid medium and then cooled to 10°C or lower to terminate the culture.

The resulting culture medium was centrifuged and the supernatant was discarded to collect the bacterial cells, then an equal volume of a citrate-phosphate buffer (pH 5.0) was added, and the bacterial cells were collected again to wash them. The above-mentioned buffer in a half volume of the original culture was added to the obtained bacterial cells to suspend them, and this suspension was used as the enzyme solution (amino acid sequence of the enzyme protein: SEQ ID NO: 1).

### (Enzymatic reaction)

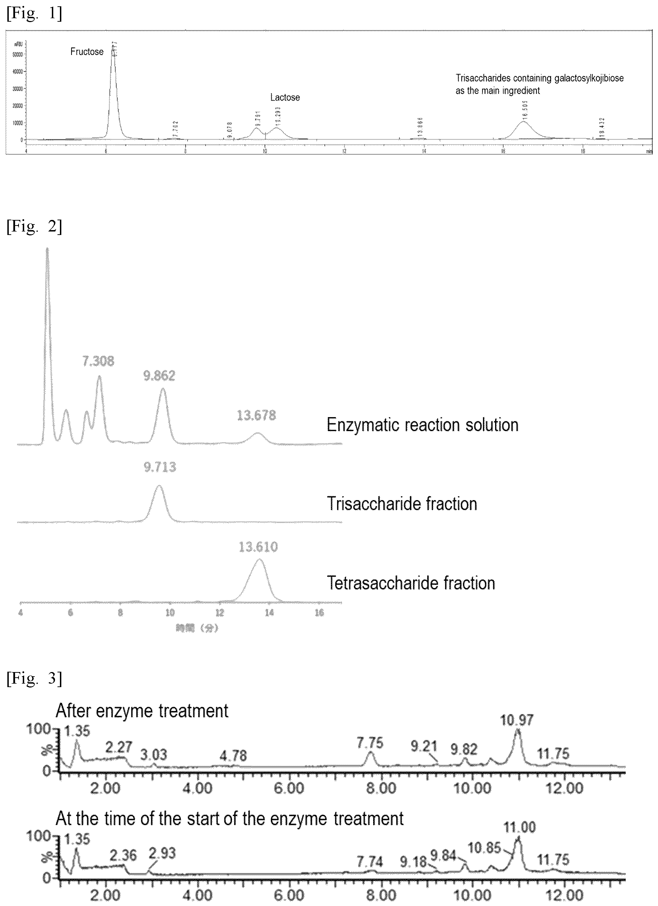

Sucrose (32 w/w %), lactose (16 w/w %), and the enzyme solution (10 w/w %) were mixed, and the reaction was allowed at 48°C for 18 hours under static conditions. The analysis results of the sugar composition after the reaction obtained by using HPLC (SUPELCO apHera^{™} NH₂ HPLC Column, mobile phase 68% acetonitrile, column temperature 35°C, flow rate 1.0 mL/min, RI detection) are shown in Fig. 1. The composition contained 25 to 30% of galactosylkojibiose, 15 to 20% of lactose, and 30 to 35% of fructose based on solid content.

### [Example 2: Production of galactosylkojibiose using whey powder]

An enzymatic reaction was performed under the same conditions as the enzymatic reaction conditions shown in Example 1, except that 20 w/w % of whey powder (Meiji Co., Ltd., containing 75% of lactose) was used instead of lactose.

A chromatogram similar to that obtained in Example 1 was obtained also when whey powder was used.

### [Example 3: Purification of trisaccharide and tetrasaccharide fractions]

The enzymatic reaction solution was passed through a carbon-Celite column, and then non-adsorbed substances were removed by passing distilled water through the column. By elution performed stepwise with ethanol solutions of different concentrations, a trisaccharide fraction containing 4'-galactosylkojibiose as the main ingredient and a tetrasaccharide fraction containing 4'-galactosylkojibiose linked with one additional molecule of glucose were obtained (Fig. 2).

### [Example 4: Lactase treatment and structural analysis of fractionation purification product]

The purified trisaccharide fraction was lyophilized and dissolved at a concentration of 10% in a phosphate-citrate buffer at pH 6.5. Then, 0.1% of lactase (product name GODO-YNL, Godo Shusei) was added to the solution, and the enzymatic reaction was allowed at 40°C. LCMS analysis using an amide column (BEH Amide, Waters) showed the formation of kojibiose (disaccharide consisting of glucoses linked through α-1,2-linkage, corresponding to an elution time of 7.75 minutes) after the reaction (Fig. 3).

Based on the above results and others, it was confirmed that the main ingredient of the trisaccharide fraction was galactosylkojibiose. Considering the activity of the enzyme, transfer to galactose residue of lactose is also possible, but surprisingly, transfer to galactose residue scarcely occurred, and galactosylkojibiose was formed (see below)).

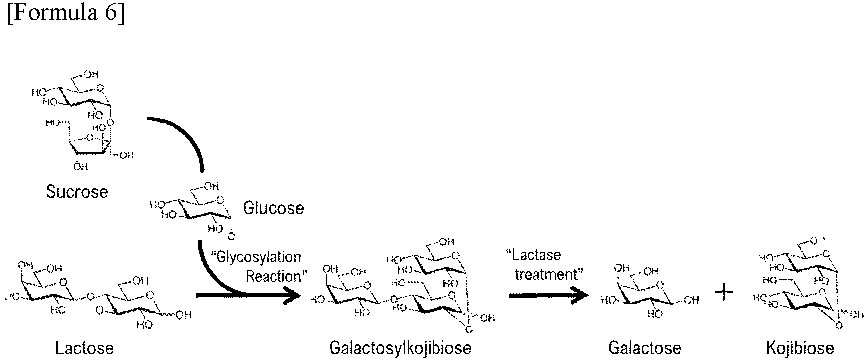

### [Example 5: Fermented milk containing galactosylkojibiose]

### (Preparation of enzyme solution)

*Lactobacillus satsumensis* JCM12392 was activation-cultured in a commercial MRS liquid medium, 1% of the cells were subcultured in the following medium, and incubated at 30°C for 27 hours while maintaining pH at constant level of 6.4 with sodium hydroxide in a jar fermenter, and then the culture system was cooled to 10°C or lower to terminate the culture.

**[Table 2]**

| Medium composition | % |
|---|---|
| Glucose | 6.5 |
| Yeast peptone HYP-A581 (Oriental Yeast) | 0.75 |
| Yeast extract Gistex LS Powder (DMS) | 1.5 |
| Yeast Rich Manganese (Oriental Yeast) | 0.2 |
| POLYALDO 10-1-O (Lonza) | 0.1 |

The resulting culture medium was centrifuged and the supernatant was discarded to concentrate the culture medium and thereby obtain a *Liquorilactobacillus satsumensis* concentrate in which cell density was concentrated 15-fold (bacterial cell concentrate producing the amino acid sequence of the enzyme protein (SEQ ID NO: 1)), and this concentrate was used as the enzyme solution.

### (Production of fermented milk containing galactosylkojibiose)

Raw material milk is prepared by mixing 500.0 g of raw milk, 53.2 g of skimmed milk powder, 23.0 g of cream, 403.6 g of tap water, and 50 g of sucrose. The raw material milk is heat-sterilized at 95°C, and the heat-sterilized raw material milk is cooled. Then, the raw material milk is inoculated with 0.5% of the *Liquorilactobacillus satsumensis* concentrate (enzyme solution), and fermented at 48°C for 18 hours under static conditions, and then *Lactobacillus bulgaricus* and *Streptococcus thermophilus* are added as a lactic acid bacterium starter. The addition amount of the lactic acid bacterium starter is 20 g. The raw material milk to which the lactic acid bacterium starter is added is filled into a cup container (capacity 100 ml, made of plastic). The raw material milk filled in the cup container is allowed to statically ferment in a fermentation chamber at 43°C until the lactic acid acidity reaches 0.7%.

### [Example 6: Evaluation of thermal stability of aqueous solution]

Buffers having various pH values (100 mM citrate-phosphate buffer for pH 3 to 6 and 100 mM phosphate buffer for pH 6 to 8) were used to dissolve the purified and fractionated galactosylkojibiose obtained in Example 3 so that the resulting solutions should have 12° Bx (12 wt %). The solutions were subjected to a heat treatment at 60°C, 80°C, 100°C, and 120°C for 20 minutes by using an autoclave, and then residual ratio of galactosylkojibiose was evaluated.

The residual ratio was calculated from the results obtained by using HPLC method performed under the following conditions.
Column: SUPELCO apHera^{™} NH₂ HPLC Column
Mobile phase: 68% acetonitrile
Column temperature: 35°C
Flow rate: 1.0 mL/min
Detection: RI

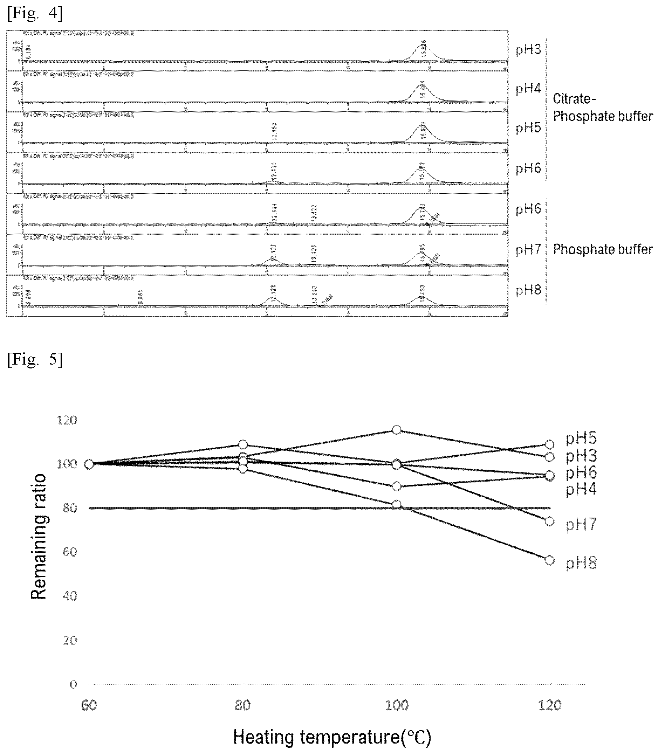

After the heat treatment at 120°C for 20 minutes, galactosylkojibiose observed at an elution time of around 15.8 minutes showed no significant change at pH 6 or lower. However, at pH 7 or higher, a new chromatographic peak, which appeared to be derived from structural change of the compound, occurred around an elution time of around 12.1 minutes (Fig. 4), simultaneously with the decay of the chromatographic peak.

In Fig. 5, there are shown the peak areas for each temperature treatment condition based on that observed with the heat treatment condition of 60°C at which decay of galactosylkojibiose was not observed at any pH value, which is taken as 100.

### [Example 7: Identification of newly generated sugar]

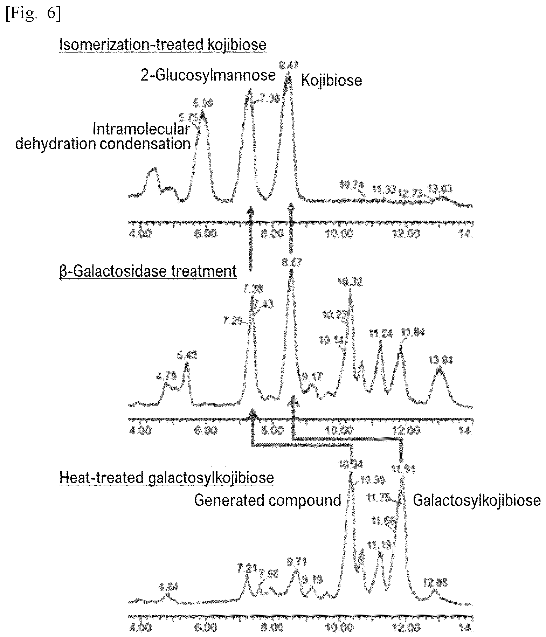

The molecular structure of the compound generated by the heat treatment (generated compound) was estimated by LC/MS method using an amide column (ACQUITY UPLC BEH Amide Column, 130 angstrom, 1.7 µm, 2.1 mm x 100 mm, Waters). Specifically, a solution of the purified and fractionated galactosylkojibiose obtained in Example 3 was heat-treated, 0.1% of β-galactosidase (lactase) GODO-YNL was added to the generated compound (Fig. 6, bottom), and the enzymatic reaction was allowed at 40°C. As a result, a new disaccharide peak, which is thought to reflect the generated compound, was observed in addition to the peak of kojibiose generated from galactosylkojibiose by hydrolysis of the galactose residue (Fig. 6, middle). The elution time of this peak coincided with that of 2-glucosylmannose obtained by isomerization of kojibiose (Fig. 6, top).

Therefore, the compound generated by the heat treatment was presumed to be glucosylepilactose (α-D-Glcp-(1→2)-[β-D-Galp-(1→4)-]D-Manp), produced by isomerization of the reducing terminal glucose of galactosylkojibiose into mannose.

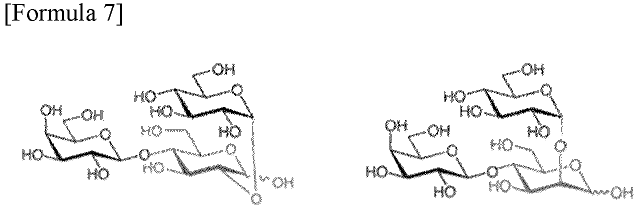

This novel compound is expected to be useful as a precursor of epilactose, which has been reported to be a promising prebiotics (Non-patent document 5: Prebiotic properties of epilactose. J Dairy Sci. 2008 Dec;91(12):4518-26).

### [Example 8: Preparation of sugar solution having adjusted pH and dried product thereof]

The pH of the composition comprising galactosylkojibiose obtained in Example 1 or 2 is confirmed, and if the pH exceeds 7, the pH is adjusted to be 7 or lower with a pH adjuster acceptable for foods. The resulting sugar solution with a pH of 7 or lower can be dried by a usual method to prepare powder.

### Industrial applicability

The present invention provides a food containing an oligosaccharide such as galactosylkojibiose and a method for producing such a food. The present invention can also be expected to realize improvement of the nutrition, ensure healthy living, and promote welfare of various people.

### [Sequences listed in Sequence Listing]

SEQ ID NOS: 1 to 8, Amino acid sequences of glycoside hydrolase
SEQ ID NOS: 9 to 21, Amino acid sequences of glycoside hydrolase (partial)

## Claims

1. A method for producing a composition comprising an oligosaccharide, the method comprising:
allowing a glucansucrase to act on a composition comprising lactose and sucrose to obtain a composition comprising galactosylkojibiose.

2. The method according to claim 1, wherein the glucansucrase is used as a lactic acid bacterium or a processed product thereof.

3. The method according to claim 2, wherein the lactic acid bacterium is a lactic acid bacterium that constitutively expresses a glucansucrase.

4. The method according to claim 1, wherein the glucansucrase is of bacterial cell-bound type.

5. The method according to claim 1, wherein the glucansucrase has a dextransucrase activity.

6. The method according to claim 2, wherein the lactic acid bacterium or the processed product thereof is any selected from the group consisting of live bacterial cells, dead bacterial cells, culture containing bacterial cells, disrupted bacterial cells, and a purified product of glucansucrase.

7. The method according to any one of claims 1 to 6, wherein the glucansucrase, or the lactic acid bacterium that expresses a glucansucrase or the processed product thereof is derived from a lactic acid bacterium belonging to the family *Lactobacillaceae.*

8. The method according to any one of claims 1 to 6, wherein the glucansucrase, or the lactic acid bacterium that expresses a glucansucrase or the processed product thereof is derived from a lactic acid bacterium belonging to the genus *Liquorilactobacillus.*

9. The method according to any one of claims 1 to 6, wherein the glucansucrase, or the lactic acid bacterium that expresses a glucansucrase or the processed product thereof is derived from a lactic acid bacterium belonging to *Liquorilactobacillus satsumensis.*

10. A method for producing a composition comprising an oligosaccharide, the method comprising:
allowing a glucansucrase consisting of the protein (A), (B), or (C) mentioned below to act on a composition comprising lactose and sucrose to obtain a composition comprising galactosylkojibiose:
(A) a protein consisting of any one of the amino acid sequences of SEQ ID NOS: 1 to 8;
(B) a protein consisting of an amino acid sequence having a high sequence identity to any one of the amino acid sequences of SEQ ID NOS: 1 to 8 and having a glucansucrase activity; or
(C) a protein consisting of an amino acid sequence derived from any one of the amino acid sequences of SEQ ID NOS: 1 to 8 by substitution, deletion, insertion, and/or addition of one or multiple amino acids and having a glucansucrase activity.

11. The method according to claim 10, wherein the glucansucrase consists of the protein (B') or (C') mentioned below:
(B') a protein consisting of an amino acid sequence having a high sequence identity to any one of the amino acid sequences of SEQ ID NOS: 1 to 8, in which the portions corresponding to the positions 450 to 467, 488 to 499, and 559 to 573 in the sequence of SEQ ID NO: 1 are identical to those of the sequence of SEQ ID NO: 1, and having a glucansucrase activity; or
(C') a protein consisting of an amino acid sequence derived from any one of the amino acid sequences of SEQ ID NOS: 1 to 8 by substitution, deletion, insertion, and/or addition of one or multiple amino acids, in which the portions corresponding to the positions 450 to 467, 488 to 499, and 559 to 573 in the sequence of SEQ ID NO: 1 are identical to those of the sequence of SEQ ID NO: 1, and having a glucansucrase activity.

12. A method for producing fermented milk containing galactosylkojibiose, the method comprising:
allowing a lactic acid bacterium that expresses a glucansucrase or a processed product thereof to act on a composition comprising lactose and sucrose to obtain a composition comprising galactosylkojibiose, and
adding a lactic acid bacterium starter to raw material milk and allowing fermentation to obtain fermented milk.

13. An enzyme agent comprising a glucansucrase consisting of the protein (A), (B), or (C) mentioned below:
(A) a protein consisting of any one of the amino acid sequences of SEQ ID NOS: 1 to 8;
(B) a protein consisting of an amino acid sequence having a high sequence identity to any one of the amino acid sequences of SEQ ID NOS: 1 to 8 and having a glucansucrase activity; or
(C) a protein consisting of an amino acid sequence derived from any one of the amino acid sequences of SEQ ID NOS: 1 to 8 by substitution, deletion, insertion, and/or addition of one or multiple amino acids and having a glucansucrase activity.

14. The enzyme agent according to claim 13, wherein the glucansucrase consists of the protein (B') or (C') mentioned below:
(B') a protein consisting of an amino acid sequence having a high sequence identity to any one of the amino acid sequences of SEQ ID NOS: 1 to 8, in which the portions corresponding to the positions 450 to 467, 488 to 499, and 559 to 573 in the sequence of SEQ ID NO: 1 are identical to those of the sequence of SEQ ID NO: 1, and having a glucansucrase activity; or
(C') a protein consisting of an amino acid sequence derived from any one of the amino acid sequences of SEQ ID NOS: 1 to 8 by substitution, deletion, insertion, and/or addition of one or multiple amino acids, in which the portions corresponding to the positions 450 to 467, 488 to 499, and 559 to 573 in the sequence of SEQ ID NO: 1 are identical to those of the sequence of SEQ ID NO: 1, and having a glucansucrase activity.

15. The enzyme agent according to claim 13 or 14, wherein the glucansucrase is contained as any selected from the group consisting of live bacterial cells, dead bacterial cells, culture containing bacterial cells, and disrupted bacterial cells of a lactic acid bacterium that expresses a glucansucrase.

16. The enzyme agent according to claim 13 or 14, which is used in The method according to any one of claims 1 to 6.

17. The enzyme agent according to claim 13 to 14, which is for producing glucosylepilactose.

18. A solution containing galactosylkojibiose, which has a pH lower than 7.

19. A composition comprising galactosylkojibiose, wherein when the composition is made into a solution having a solid content concentration of 10 to 80%, the solution has a pH lower than 7.

20. The composition according to claim 19, which contains 10% or more of fructose based on solid content.

21. The solution according to claim 18 or the composition according to claim 19 or 20, which is obtainable by the method according to any one of claims 1 to 6 and 10 to 12.

22. A composition comprising galactosylkojibiose, which has the following characteristics:
(1) the composition contains 15 to 40% of galactosylkojibiose based on solid content,
(2) the composition contains 15 to 45% of fructose based on solid content, and
(3) when the composition is made into a solution having a solid content concentration of 12% and heated at 120°C for 20 minutes, galactosylkojibiose remaining ratio in the solution is 80% or higher.

23. Glucosylepilactose or a composition comprising glucosylepilactose.

24. The method according to any one of claims 1 to 6 and 10 to 12, which further comprises the step of adjusting pH of the resulting composition to be lower than 7.

25. The method according to any one of claims 1 to 6 and 10 to 12, wherein the composition comprising an oligosaccharide contains galactosylkojibiose and fructose.

26. The method according to any one of claims 1 to 6 and 10 to 12, wherein the composition comprising an oligosaccharide contains 10% or more, but no more than 100% of galactosylkojibiose based on solid content.

27. The method according to any one of claims 1 to 6 and 10 to 12, wherein the composition comprising an oligosaccharide contains 1% or more of fructose based on solid content.

28. The method according to any one of claims 1 to 6 and 10 to 12, wherein mass ratio of galactosylkojibiose and lactose (galactosylkojibiose:lactose) in the composition comprising an oligosaccharide is 15 to 40:15 to 30.

29. The method according to any one of claims 1 to 6 and 10 to 12, wherein the composition comprising an oligosaccharide contains glucosylepilactose.

30. The method according to any one of claims 1 to 6 and 10 to 12, which is for producing the composition comprising an oligosaccharide according to any one of claims 18 to 23.
